# EUROPEAN PATENT SPECIFICATION

(11) **EP 3 124 619 B1**
(45) Date of publication and mention of the grant of the patent: **06.03.2019**
(21) Application number: 15179348.6
(22) Date of filing: 31.07.2015
(51) Int. Cl.: C12Q 1/6883

(54) **REAGENTS, METHOD AND KIT FOR ACROSS AND WITHIN DOG BREED GLAUCOMA DIAGNOSIS**
REAGENZIEN, VERFAHREN UND KIT ZUR GLAUKOMDIAGNOSE ÜBER HUNDERASSEN HINWEG UND INNERHALB VON HUNDERASSEN
RÉACTIFS, PROCÉDÉ ET KIT POUR LE DIAGNOSTIC DU GLAUCOME DE RACES CANINES

(43) Date of publication of application: 01.02.2017
(73) Proprietor: Menicon Co., Ltd, Nagoya 460-0006 (JP)
(72) Inventor: TCHEDRE, Kissaou, 1205 Geneva (CH); LAMRANI, Mouad, 1205 Geneva (CH); OMORI, Tetsushi, 1205 Geneva (CH)
(74) Representative: Cabinet Plasseraud

(56) References cited:
- WO-A1-2013/173637
- US-A1- 2014 141 432
- NOBUYUKI KANEMAKI ET AL: "Dogs and Humans Share a Common Susceptibility Gene SRBD1 for Glaucoma Risk", PLOS ONE, vol. 8, no. 9, 11 September 2013 (2013-09-11), page e74372, XP055220659, DOI: 10.1371/journal.pone.0074372
- THE WRITING COMMITTEE FOT THE NORMAL TENSION GLAUCOMA GENETIC STUDY ET AL: "Genome-wide Association Study of Normal Tension Glaucoma: Common Variants in SRBD1 and ELOVL5 Contribute to Disease Susceptibility", OPHTHALMOLOGY, J. B. LIPPINCOTT CO., PHILADELPHIA, PA, US, vol. 117, no. 7, 1 July 2010 (2010-07-01), pages 1331-1338.e5, XP027113427, ISSN: 0161-6420 [retrieved on 2010-06-30]

## Description

### Background of the Invention

Glaucoma is a degenerative optic neuropathy associated with elevated intraocular pressure. If the condition becomes chronic and persists without treatment, it eventually causes permanent damage to the optic nerve, resulting in irreversible vision field loss, which can progress to blindness. Glaucoma affects not only humans but also many other mammals, including dogs.

Canine glaucoma can be divided into two main categories, primary glaucoma and secondary glaucoma, based on the causative factors involved. Primary glaucoma is associated with inherent abnormalities within the aqueous outflow pathways of the eye, resulting in sustained increase in intraocular pressure. Although primary glaucoma has been identified in almost every breed of dog, certain breeds are predisposed to getting primary glaucoma, including the Cocker Spaniels, many of the Terrier breeds such as the Jack Russell Terrier, Northern breeds such as the Siberian Husky and Elkhound, and other breeds such as the Basset Hound, Chow Chow, Shar Pei, Shih Tzu, Beagles, Poodles, and Dalmatians. Secondary glaucoma is a complication of another eye disease that causes decreased drainage of the eye such as, for example, uveitis (inflammation inside the eye), cataract, displacement of the lens (subluxation or luxation), or trauma to the eye. While treatment of secondary glaucoma is directed toward the underlying eye problem, the goal of treating early forms of primary glaucoma is to maintain a normal intraocular pressure and to preserve vision. Thus, although there is no prophylactic method for glaucoma, the progress of the disease can be inhibited, or at least delayed, when it is found at an early stage. However, in many cases, glaucomatous dogs are brought to veterinary hospitals when their symptoms have advanced, and when response to medical and surgical treatment is often limited and short-lived.

Indeed, an eye affected with glaucoma may sometimes be red, swollen, and/or sore or become clouded in appearance, however, canine glaucoma often goes unnoticed until it is in a more severe state. There are rarely any symptoms in the early stages of the disease, so regular eye checks by qualified veterinary professionals are important. Currently, the diagnosis of glaucoma can be made only by a veterinary eye examination using three different methods: tonometry (which measures the intraocular pressure), gonioscopy (a diagnostic procedure which examines the angle of the anterior chamber of the eye) and ophthalmoscopy (which evaluates the retina and particularly the optic nerve). However, these methods are only efficient on the late stage of glaucoma development. Therefore, there is an urgent need for tests allowing early diagnosis of glaucoma.

Glaucoma is known as a genetically heterogeneous disease, and many genes, including *SRBD1, ELOVL5, CYP1B1, MYOC, OPTN,* and *OPTC* have been reported to be linked to primary open angle glaucoma (POAG) and primary congenital glaucoma (PCG) in humans and/or in dogs. Kutchey and coworkers have described the Gly661Arg variant in *ADAMTS10* as the candidate disease-causing variant for POAG in Beagles (PLoS Genet., 2011, 7: e1001306). *SRBD1* polymorphisms have been reported to play an important role in glaucoma pathology in Shiba-Inus and in Shih-Tzus (Kanemaki et al., PLoS one, 2013, 8(9): e74372 and US 2014/0141432). A missense mutation was discovered in *ADAMTS10* in Norwegian Elkhound primary glaucoma (Ahonen et al., PLoS One, 2014, 9(11): e11941). It was recently demonstrated that variants in NEB are linked to the development of familial POAG in Basset Hounds (Dina et al., PLoS one, 2015, 10(5): e0126660).

In spite of the progress made in developing genetic testing for canine glaucoma, there still remains a need in the art for new methods which allow the diagnosis of glaucoma earlier and more reliably and which can be applied to a wide variety of dog breeds.

### Summary of the Invention

The present Inventors have analyzed a glaucomatous dog population and a normal/healthy (*i.e.,* non-glaucomatous) dog population using direct sequencing, and identified two Single Nucleotide Polymorphisms (SNPs) within the dog *SRBD1* gene, with International SNP Numbers rs9172407 and rs22115601. In contrast to other known SNPs associated with canine glaucoma, rs9172407 and rs22115601 were found to be especially effective for within and across dog breed glaucoma diagnosis (see Examples section below).

Accordingly, in a first aspect, the present invention relates to an *in vitro* method for identifying a subject at risk of having or developing canine glaucoma, said method comprising a step of: determining, in a biological sample obtained from said subject, the identity, presence or absence of a variant allele of single nucleotide polymorphism (SNP) rs22115601, which is located at nucleotide position 401 of the flanking sequence SEQ ID NO: 2.

In certain embodiments, in *an vitro* method of the invention, the subject is identified as being at risk of having or developing canine glaucoma if the variant allele of rs22115601 is T at nucleotide position 401 of the flanking sequence SEQ ID NO: 2.

In another aspect, the present invention relates to an *in vitro* method for identifying a subject at risk of having or developing canine glaucoma, said method comprising a step of: determining, in a biological sample obtained from said subject, the identity, presence or absence of a variant allele of a SNP located within the *SRBD1* gene, wherein said SNP is selected from the group consisting of rs9172407, rs22115601, and combination thereof, wherein the subject belongs to any one of the dog breeds from the group consisting of Akita, Alaskan Malamute, Basset Hound, Beagle, Border Collie, Boston Terrier, Bouvier des Flandres, Brittany Spaniel, Cairn Terrier, Cardigan Welsh, Corgi, Chihuahua, Chow Chow, American Cocker Spaniel, Dachshund, Dalmatian, Great Dane, Dandie Dinmont Terrier, Elkhound, English Cocker Spaniel, English Springer Spaniel, German Shepherd, Giant Schnauzer, Greyhound, Irish Setter, Italian Greyhound, Jack Russell Terrier, Keeshond, Lakeland Terrier, Maltese, Miniature Pinscher, Miniature Schnauzer, Norfolk Terrier, Norwegian Elkhound, Norwich Terrier, Poodle, Samoyed, Scottish Terrier, Sealyham Terrier, Shar Pei, Siberian Husky, Skye Terrier, Smooth Fox Terrier, Tibetan Terrier, Welsh Springer Spaniel, Welsh Terrier, West Highland White Terrier, Wire Fox Terrier, French Bulldog, Boxer, Bichon, German Shepherd, Short-Haired Dachshund, Golden Retriever, Border Collie, Newfoundland, Griffon Vendéen, Australian Shepherd, Long-Haired Dachshund, Groenendael, Doberman, Beauceron, Weimaraner, Bull Terrier, Hovawart, Polish Lowland Sheepdog, Labrador, Great Dane, Pointer, English Setter, Gordon Setter, Korthals Griffon, American Cocker, Jagdterrier, Brittany, Italian Greyhound, Labrador, Pomeranian, Lhassa apso, Cavalier King Charles Spaniel, Tervuren, Bouvier des Flandres, Staffordshire Bull Terrier, Flat-Coated Retriever, Schnauzer, Eurasier,Yorkshire Terrier, Pinscher, Leonberger, and West Highland White Terrier, and wherein the subject is identified as being at risk of having or developing canine glaucoma if the variant allele of rs9172407 is T at nucleotide position 401 of the flanking sequence SEQ ID NO: 1 and/or if the variant allele of rs22115601 is T at nucleotide position 401 of the flanking sequence SEQ ID NO: 2.

In certain embodiments, in an *in vitro* method according to the invention, the subject is a dog which does not exhibit any symptoms of glaucoma.

In certain embodiments, in an *in vitro* method according to the invention, the biological sample is selected from the group consisting of blood, semen, saliva, tears, urine, fecal material, sweat, buccal cells, skin, hair and other nucleic acid-containing tissue. For example, the biological sample may comprise genomic DNA.

In another aspect, the present invention relates to a reagent for use in a method for identifying a subject at risk of having or developing canine glaucoma, wherein said reagent comprises a pair of primers capable of amplifying at least part of a nucleic acid region containing SNP rs22115601 and optionally a probe capable of specifically hybridizing to at least part of a nucleic acid region containing SNP rs22115601, wherein said pair of primers comprises a forward primer consisting of the sequence set forth in SEQ ID NO: 5 and a reverse primer consisting of the sequence set forth in SEQ ID NO: 6.

In certain embodiments, in the reagent according to the invention, the probe capable of specifically hybridizing to at least part of a nucleic acid region containing SNP rs22115601 can specifically hybridize a nucleic acid region of the flanking sequence SEQ ID NO: 2.

In certain embodiments, the reagent further comprises a pair of primers capable of amplifying at least part of a nucleic acid region containing SNP rs9172407 and optionally a probe capable of specifically hybridizing to at least part of a nucleic acid region containing SNP rs9172407, wherein said pair of primers comprises a forward primer consisting of the sequence set forth in SEQ ID NO: 3 and a reverse primer consisting of the sequence set forth in SEQ ID NO: 4.

In certain embodiments, the probe capable of specifically hybridizing to at least part of a nucleic acid region containing SNP rs9172407 can specifically hybridize a nucleic acid region of the flanking sequence SEQ ID NO: 1.

In certain embodiments, the probe capable of specifically hybridizing to at least part of a nucleic acid region containing SNP rs22115601 is labeled with a detectable agent or moiety.

In certain embodiments, the probe capable of specifically hybridizing to at least part of a nucleic acid region containing SNP rs22115601 is immobilized on a solid support.

In certain embodiments, the probe capable of specifically hybridizing to at least part of a nucleic acid region containing SNP rs9172407 is labeled with a detectable agent or moiety.

In certain embodiments, the probe capable of specifically hybridizing to at least part of a nucleic acid region containing SNP rs9172407 is immobilized on a solid support.

In yet another aspect, the present invention relates to a kit for identifying a subject at risk of having or developing canine glaucoma comprising a reagent described herein.

In certain embodiments, a kit of the invention further comprises instructions for performing an *in vitro* method described herein.

In certain embodiments, a kit of the invention further comprises amplification reagents.

In certain embodiments, a kit of the invention further comprises at least one buccal swab brush.

These and other objects, advantages and features of the present invention will become apparent to those of ordinary skill in the art having read the following detailed description of the preferred embodiments.

### Definitions

Throughout the description, several terms are employed that are defined in the following paragraphs.

The terms ***"subject"*** and ***"dog"*** are used herein interchangeably. These terms do not denote a particular age or a particular breed. As used herein, the term ***"glaucomatous dog"*** refers to a dog which has been clinically diagnosed with glaucoma.

The terms ***"single nucleotide polymorphism"*** and ***"SNP"*** are used herein interchangeably. They refer to a polymorphism where each allele differs by the replacement of a single nucleotide in the DNA sequence of the allelic gene. In some cases, the single nucleotide change can alter the structure and function of the corresponding gene product (*i.e.,* protein). However, SNPs are not always associated with genes; they can also occur in non-coding regions of DNA. For most SNPs, only two of the four possible nucleotides (adenosine (A), thymidine (T), guanosine (G), or cytidine (C)) are observed. SNPs can be bi-, tri-, or tetra-allelic polymorphisms. The SNPs of the present invention are bi-allelic polymorphisms.

As used herein, the term ***"allele"*** refers to one of a pair or a series of genetic variants of a polymorphism at a specific genomic location. The terms ***"polymorphism"*** and ***"allelic variant"*** refer to an alteration in the normal (*i.e.,* wild-type) sequence of a gene. The terms ***"canine glaucoma allele"*** and ***"canine glaucoma risk allele"*** are used herein interchangeably and refer to the allele that is associated with canine glaucoma or to a risk (*i.e.,* increased probability) of developing canine glaucoma. The term ***"canine glaucoma protective allele",*** as used herein, refers to the allele that is associated with no risk or an non-significant risk or a decreased risk of developing canine glaucoma.

As used herein, the term ***"genotype"*** refers to the diploid combination of alleles for a given genetic polymorphism. A homozygous subject carries two copies of the same allele and a heterozygous subject carries two different alleles.

The terms ***"nucleic acid", "nucleic acid molecule",*** and ***"polynucleotide"*** are used herein interchangeably. They refer to a deoxyribonucleotide or ribonucleotide polymer in either single-stranded or double-stranded form, and unless otherwise stated, encompass known analogs of natural nucleotides that can function in a similar manner as naturally occurring nucleotides. The terms encompass nucleic acid-like structures with synthetic backbones, as well as amplification products.

The term ***"oligonucleotide",*** as used herein, refers to a short string of a deoxyribonucleotide or ribonucleotide polymer. Oligonucleotides can be used as amplification primers and/or detection probes. Such short stretches of nucleic acid sequences are often chemically synthesized. As will be appreciated by those skilled in the art, the length of an oligonucleotide can vary widely, often depending on its intended function or use. Generally, oligonucleotides comprise between about 5 and about 150 nucleotides, preferably between about 15 and about 100 nucleotides, more preferably between about 15 and about 50 nucleotides.

The term ***"isolated"*** when referring to an oligonucleotide means an oligonucleotide which, by virtue of its origin or manipulation, is separated from at least some of the components with which it is naturally associated or with which it is associated when initially obtained or prepared. By "isolated", it is alternatively or additionally meant that the oligonucleotide of interest is produced or synthesized by the hand of man.

The term ***"hybridization"*** refers to the formation of complexes between nucleotide sequences which are sufficiently complementary to form complexes *via* Watson-Crick base pairing or non-canonical base pairing. When a primer "hybridizes" with a target sequence (template), such complexes (or hybrids) are sufficiently stable to serve the priming function required by, *e.g.,* the DNA polymerase, to initiate DNA synthesis. It will be appreciated that hybridizing sequences need not have perfect complementarity to provide stable hybrids. In many situations, stable hybrids will form where fewer than about 10% of the bases are mismatches. Those skilled in the art understand how to estimate and adjust the stringency of hybridization conditions such that sequences having at least a desired level of complementarity will stably hybridize, while those having lower complementarity will not. For examples of hybridization conditions and parameters see, *e.g.,* J. Sambrook et al., "Molecular Cloning: A Laboratory Manual", 2012, 4th Ed., Cold Spring Harbor Press: Plainview, NY; F.M. Ausubel, "Current Protocols in Molecular Biology", 1994, John Wiley & Sons: Secaucus, NJ. As used herein, the term ***"specifically hybridizes"*** means that cross-hybridization with DNA other than the DNA having the nucleotide sequence of the region comprising a polymorphic site of interest (*e.g.,* a SNP of the present invention) is not significantly produced under ordinary hybridization conditions, preferably under stringent hybridization conditions (see, for example, the conditions disclosed in Sambrook et al., "Molecular Cloning: A Laboratory Manual", 2012, 4th Ed., Cold Spring Harbor Press: Plainview, NY).

The expression ***"sequence complementary to"*** a given nucleotide sequence is intended to mean a sequence which forms, by hybridization, a stable duplex with said nucleotide sequence. The expression "sequence complementary to" denotes both the complementary sequence presented in the 3'→5' direction and the complementary sequence presented in the 5'→3' direction (*i.e.* the reverse complementary sequence). In certain cases, the hybridization is perfect, *i.e.* the sequences are totally complementary. Thus, as used herein, the expression ***"the sequence complementary to the sequence SEQ ID NO: X**"* is the nucleotide sequence which is perfectly or totally complementary to SEQ ID NO: X.

As used herein, the term ***"amplification"*** refers to a method or process that increases the representation of a population of specific nucleic acid sequences in a sample. Amplification methods (such as polymerase chain reaction or PCR) are known in the art and are discussed in more detail below.

The terms ***"target sequence"*** and ***"target nucleic acid**"* are used herein interchangeably. They refer to a nucleic acid sequence, the presence or absence of which is desired to be detected. In the context of the present invention, a target sequence preferably includes a nucleic acid sequence to which oligonucleotide primers will complex. The target sequence may also include a probe-hybridizing region with which a probe will form a stable hybrid under desired conditions. As will be recognized by one of ordinary skill in the art, a target sequence may be single-stranded or double-stranded. In the context of the present invention, target sequences of interest encompass a SNP of the invention.

The terms ***"primer"*** and ***"amplification primer"*** are used herein interchangeably. They refer to an isolated oligonucleotide which is capable of acting as a point of initiation of synthesis of a primer extension product that is a complementary strand of DNA, when placed under suitable conditions (*e.g.,* buffer, salt, temperature and pH) in the presence of nucleotides and an agent for nucleic acid polymerization (*e.g.,* a DNA-dependent or RNA-dependent polymerase). The primer is preferably single-stranded for maximum efficiency in amplification, but may alternatively be double-stranded. If double-stranded, the primer may first be treated (*e.g.,* denatured) to allow separation of its strands before being used to prepare extension products. Such a denaturation step is typically performed using heat, but may alternatively be carried out using alkali, followed by neutralization. A typical primer contains about 10 to about 50 nucleotides in length of a sequence substantially complementary to the target sequence. However, a primer can also contain additional sequences. For example, amplification primers used in Strand Displacement Amplification (SDA) preferably include a restriction endonuclease recognition at site 5' to the target binding sequence (see, for example, U.S. Pat. Nos 5,270,184 and 5,455,166). Nucleic Acid Sequence Based Amplification (NASBA), and Transcription-Mediated Amplification (TMA) primers preferably include an RNA polymerase promoter linked to the target binding sequence of the primer. Methods for linking such specialized sequences to a binding target sequence for use in a selected amplification reaction are well-known in the art.

The terms ***"forward primer"*** and ***"forward amplification primer"*** are used herein interchangeably, and refer to a primer that hybridizes (or anneals) with the target (template strand). The terms ***"reverse primer"*** and ***"reverse amplification primer"*** are used herein interchangeably, and refer to a primer that hybridizes (or anneals) to the complementary target strand. The forward primer hybridizes with the target sequence 5' with respect to the reverse primer.

The term ***"amplification conditions",*** as used herein, refers to conditions that promote annealing and/or extension of primer sequences. Such conditions are well-known in the art and depend on the amplification method selected. Thus, for example, in a PCR reaction, amplification conditions generally comprise thermal cycling, *i.e.,* cycling of the reaction mixture between two or more temperatures. In isothermal amplification reactions, amplification occurs without thermal cycling although an initial temperature increase may be required to initiate the reaction. Amplification conditions encompass all reaction conditions including, but not limited to, temperature and temperature cycling, buffer, salt, ionic strength, pH and the like.

As used herein, the term ***"amplification reaction reagents"*** refers to reagents used in nucleic acid amplification reactions and may include, but are not limited to, buffers, reagents, enzymes having reverse transcriptase and/or polymerase activity or exonuclease activity; enzyme cofactors such as magnesium or manganese; salts; and deoxynucleotide triphosphates (dNTPs) such as deoxyadenosine triphosphate (dATP), deoxyguanosine triphosphate (dGTP), deoxycytidine triphosphate (dCTP), deoxythymidine triphosphate (dTTP) and deoxyuridine triphosphate (dUTP). Amplification reaction reagents may readily be selected by one skilled in the art depending on the amplification method used.

The terms *"probe"* and ***"detection probe"*** are used herein interchangeably and refer to an oligonucleotide capable of selectively hybridizing to at least a portion of a target sequence under appropriate conditions (*e.g.*, a portion of a target sequence that has been amplified). In general, a probe sequence is identified as being either "complementary" (*i.e.,* complementary to the coding or sense strand (+)), or "reverse complementary" (*i.e.,* complementary to the anti-sense strand (-)). A detection probe may be labeled with a detectable agent or moiety.

The terms ***"labeled"*** and ***"labeled with a detectable agent or moiety"*** are used herein interchangeably to specify that an entity (*e.g.,* an oligonucleotide detection probe) can be visualized, for example following binding to another entity (*e.g.,* an amplification reaction product or amplicon). Preferably, the detection agent or moiety is selected such that it generates a signal which can be measured and whose intensity is related to (*e.g.*, proportional to) the amount of bound entity. A wide variety of systems for labeling and/or detecting nucleic acid molecules are well-known in the art. Labeled nucleic acids can be prepared by incorporation of, or conjugation to, a label that is directly or indirectly detectable by spectroscopy, photochemical, biochemical, immunochemical, electrical, optical, chemical or other means. Suitable detectable agents include, but are not limited to, radionuclides, fluorophores, chemiluminescent agents, microparticles, enzymes, colorimetric labels, magnetic labels, haptens, Molecular Beacons, and aptamer beacons.

The terms ***"fluorophore", "fluorescent moiety"*** and ***"fluorescent dye"*** are used herein interchangeably. They refer to a molecule that absorbs a quantum of electromagnetic radiation at one wavelength, and emits one or more photons at a different, typically longer, wavelength in response. Numerous fluorescent dyes of a wide variety of structures and characteristics are suitable for use in the practice of the invention. Methods and materials are known for fluorescently labeling nucleic acid molecules (see, for example, R.P. Haugland, "Molecular Probes: Handbook of Fluorescent Probes and Research Chemicals 1992-1994", 5th Ed., 1994, Molecular Probes, Inc.). Preferably, a fluorescent moiety absorbs and emits light with high efficiency (*i.e.,* has a high molar absorption coefficient at the excitation wavelength used, and a high fluorescence quantum yield), and is photostable (*i.e.,* does not undergo significant degradation upon light excitation within the time necessary to perform the analysis). Rather than being directly detectable themselves, some fluorescent dyes transfer energy to another fluorescent dye in a process called fluorescent resonance energy transfer (FRET), and the second dye produces the detected signal. Such FRET fluorescent dye pairs are also encompassed by the term "fluorescent moiety". The use of physically linked fluorescent reporter/quencher moiety is also within the scope of the present invention. In these embodiments, when the fluorescent reporter and quencher moiety are held in close proximity, such as at the ends of a nucleic acid probe, the quencher moiety prevents detection of a fluorescent signal from the reporter moiety. When the two moieties are physically separated, such as, for example, after cleavage by a DNA polymerase, the fluorescent signal from the reporter moiety becomes detectable.

The term ***"directly detectable",*** when used herein in reference to a label or detectable moiety, means that the label or detectable moiety does not require further reaction or manipulation to be detectable. For example, a fluorescent moiety is directly detectable by fluorescence spectroscopy methods. The term ***"indirectly detectable",*** when used herein in reference to a label or detectable moiety, means that the label or detectable moiety becomes detectable after further reaction or manipulation. For example, a hapten becomes detectable after reaction with an appropriate antibody attached to a reporter, such as a fluorescent dye.

The term ***"biological sample"*** is used herein in its broadest sense. In the practice of the present invention, a biological sample is generally obtained from a dog to be tested. However, the sample may also be an archival sample with known diagnosis, treatment, and/or outcome history. A sample may be any biological fluid and/or tissue extracts (such as homogenates or solubilized tissues) with which genetic polymorphisms may be detected. Tissue extracts are obtained routinely from tissue biopsy and autopsy material. Examples of bodily fluids suitable for use in the practice of the present invention include, but are not limited to, blood, serum, plasma, urine, saliva, phlegm, gastric juices, semen, tears, sweat, etc... Preferably, the biological sample is a buccal swab. The term ***"buccal swab",*** also known as ***"buccal smear",*** refers to a DNA collection process involving cells taken from the cheek. The term "biological sample" also encompasses any material derived by processing a biological sample. Derived materials include, but are not limited to, cells (or their progeny) isolated from the sample, as well as nucleic acid molecules (DNA and/or RNA) extracted from the sample. Processing of a biological sample may involve one or more of: filtration, extraction, concentration, inactivation of interfering components, addition of reagents and the like.

The terms ***"risk", "susceptibility", "propensity"*** and ***"predisposition"*** are used herein interchangeably, and refer to the probability of developing canine glaucoma. The term *"**at risk of having or developing canine glaucoma**"**,*** when used herein to characterize a dog, refers to a dog that exhibits a higher probability than the general dog population to develop canine glaucoma.

The term ***"treatment"*** is used herein to characterize a method that is aimed at delaying or preventing the onset of a disease or condition (here canine glaucoma); or slowing down or stopping the progression, aggravation, or deteriorations of the symptoms of the condition; or bringing about ameliorations of the symptoms of the condition; or curing the condition.

The terms ***"approximately"*** and ***"about",*** as used herein in reference to a number, generally include numbers that fall within a range of 10% in either direction of the number (greater than or less than the number) unless otherwise stated or otherwise evident from the context (except where such number would exceed 100% of a possible value).

### Detailed Description of Certain Preferred Embodiments

As already indicated above, two SNPs, which were found to be highly effective for identifying dogs at risk of developing glaucoma are disclosed herein.

### I - Single Nucleotide Polymorphisms Associated with Canine Glaucoma

The two SNPs are located within the canine *SRBD1* (S1 RNA binding domain 1) gene. This gene is located on the reverse strand of chromosome 10, and is approximately 186 kb, including approximately 3 kb of the exon region (ensembl Accession Number: ENSCAFT00000049409 - see http://asia.ensembl.org/Canis_familiaris/Transcript/Exons?db=coreg=ENSCAFG00000 002554r=10:47923593-47924593t=ENSCAFT00000049409). Currently, the exact function of *SRBD1* in humans and in dogs remains unknown.

Both SNPs are already known. However, it is the first time that they are both identified as being associated with canine glaucoma risk. In the present Description, SNPs are defined by their SNP ID number attributed by the dbSNP, *i.e.,* the Single Nucleotide Polymorphism Database of the NCBI (National Center for Biotechnology Information). Nucleotide positions provided by the dbSNP are based on the genome database build2.1 of NCBI.

The first SNP has dbSNP ID Number: rs9172407. It is characterized as follows: located on Chromosome 10; position (CanFam3.1): 47937246; HGVS Name: ENSCAFT00000049409.1:c.51+1746G>A; Risk allele: G - ; SNP type: Intron Variant). rs9172407 is located at nucleotide position 401 in the flanking sequence SEQ ID NO: 1, wherein ambiguity code Y is C or T.
SEQ ID NO: 1

The canine glaucoma risk allele for rs9172407 is T at nucleotide position 401 of the flanking sequence SEQ ID NO: 1. Therefore, the subject tested is identified as being at risk of having or developing canine glaucoma if the variant allele of rs9172407 is T at nucleotide position 401 of the flanking sequence SEQ ID NO: 1. Alternatively, or additionally, the subject tested is identified as being at risk of having or developing canine glaucoma if genotype C/T or genotype T/T is determined for SNP rs9172407.

As will be acknowledged by one skilled in the art, a similar reasoning may be applied to the nucleotide sequence that is complementary to the flanking sequence SEQ ID NO: 1. Thus, is described herein a method for identifying a dog at risk of having or developing glaucoma utilizing primers and/or probes designed using the sequence complementary to SEQ ID NO: 1 to determine the identity, presence or absence of SNP rs9172407.

The second SNP has dbSNP ID Number: rs22115601. It is characterized as follows: located on Chromosome 10; position (CanFam3.1): 47737941, HGVS Name: 10:g.47737941C >T; Risk allele: T, SNP Type: Downstream gene variant). rs22115601 is located at nucleotide position 401 in the flanking sequence SEQ ID NO: 2, wherein ambiguity code Y is C or T.
SEQ ID NO: 2

The canine glaucoma risk allele for rs22115601 is T at nucleotide position 401 of the flanking sequence SEQ ID NO: 2. Therefore, the subject tested is identified as being at risk of having or developing canine glaucoma if the variant allele of rs22115601 is T at nucleotide position 401 of the flanking sequence SEQ ID NO: 2. Alternatively, or additionally, the subject tested is identified as being at risk of having or developing canine glaucoma if one of: genotype C/T and genotype T/T is determined for SNP rs22115601.

As will be acknowledged by one skilled in the art, a similar reasoning may be applied to the nucleotide sequence that is complementary to the flanking sequence SEQ ID NO: 2. Thus, is also described herein a method for identifying a dog at risk of having or developing glaucoma utilizing primers and/or probes designed using the sequence complementary to SEQ ID NO: 2 to determine the identity, presence or absence of SNP rs22115601.

### II - Methods of Identifying Canine Glaucoma Risk

Thus, is described herein a method for identifying a dog at risk of having or developing glaucoma. The method generally comprises providing a biological sample obtained from the dog to be tested, and determining, in the biological sample, the identity, presence or absence of a variant allele of a SNP disclosed herein, *i.e.,* SNP rs9172407 and SNP rs22115601. The method may include the identification of only one SNP or of both SNPs. For each SNP, either the sense strand or the anti-strand of the relevant gene (*i.e.,* the canine *SRBD1* gene) may be analyzed. The homozygosity or heterozygosity of one SNP or of both SNPs may be determined.

### Dogs and Dog Breeds

As already mentioned above, one of the major advantages of the method described herein is that it can be used for predicting susceptibility to glaucoma in dogs irrespective of their breed (*i.e.,* in purebred dogs). The method may also be applied to dog hybrids and crossbreeds as well as to mixed-breed dogs. The dogs to be tested may be companion dogs (*i.e.,* pets) or working dogs (*e.g.,* guide dogs for the visually impaired, therapy dogs, search and rescue dogs, herding dogs, pastoral dogs, sled dogs, guard dogs, hunting dogs, detection dogs that help detect illegal substances, bombs, chemicals and many other substances, military and police dogs, and the like).

The dog to be tested may be from a dog breed that is known to be predisposed to getting primary glaucoma. Examples of dog breeds that are predisposed to getting primary glaucoma, include, but are not limited to, Akita, Alaskan Malamute, Basset Hound, Beagle, Border Collie, Boston Terrier, Bouvier des Flandres, Brittany Spaniel, Cairn Terrier, Cardigan Welsh, Corgi, Chihuahua, Chow Chow, American Cocker Spaniel, Dachshund, Dalmatian, Great Dane, Dandie Dinmont Terrier, Elkhound, English Cocker Spaniel, English Springer Spaniel, German Shepherd, Giant Schnauzer, Greyhound, Irish Setter, Italian Greyhound, Jack Russell Terrier, Keeshond, Lakeland Terrier, Maltese, Miniature Pinscher, Miniature Schnauzer, Norfolk Terrier, Norwegian Elkhound, Norwich Terrier, Poodle (Toy/Miniature), Samoyed, Scottish Terrier, Sealyham Terrier, Shar Pei, Siberian Husky, Skye Terrier, Smooth Fox Terrier, Tibetan Terrier, Welsh Springer Spaniel, Welsh Terrier, West Highland White Terrier, and Wire Fox Terrier.

The dog to be tested may be from a dog breed that is not known to be predisposed to getting primary glaucoma. Examples of such dog breeds include, but are not limited to, French Bulldog, Boxer, Bichon, German Shepherd, Short-Haired Dachshund, Golden Retriever, Border Collie, Newfoundland, Griffon Vendéen, Australian Shepherd, Long-Haired Dachshund, Groenendael (or Belgian Sheepdog), Doberman, Beauceron, Weimaraner, Bull Terrier, Hovawart, Polish Lowland Sheepdog, Labrador, Great Dane, Pointer, English Setter, Gordon Setter, Korthals Griffon, American Cocker, Jagdterrier, Brittany, Italian Greyhound, Labrador, Pomeranian, Lhassa apso, Cavalier King Charles Spaniel, Tervuren, Bouvier des Flandres, Staffordshire Bull Terrier, *Flat-Coated Retriever,* Schnauzer, Eurasier,Yorkshire Terrier, Pinscher, Leonberger, and West Highland White Terrier (or Westie).

Since a method described herein may be used before the onset of glaucoma, dogs that can be tested using a method described herein may be healthy dogs that exhibit no symptoms of glaucoma, such as red eye, swollen eye, sore eye, clouded eye and/or increased intraocular pressure.

### Biological Samples

A method described herein comprises a step of providing a biological sample obtained from a dog to be tested. It may be applied to the study of any biological sample allowing the identity, presence or absence of the SNPs disclosed herein to be assessed. Samples that are suitable for use in a method described herein contain genetic material, *e.g.,* genomic DNA (gDNA). Genomic DNA is typically extracted from biological samples such as blood or mucosal scrapings of the lining of the mouth, but can be extracted from other biological samples including urine. The sample itself will typically consist of nucleated cells (*e.g.,* blood cells or buccal cells) or tissue removed from the subject. Methods and reagents are known in the art for obtaining, processing and analyzing samples. The sample may be obtained with the assistance of a veterinary professional, *e.g.,* to draw blood. Alternatively, the sample may be obtained without the assistance of a veterinary professional, *e.g.,* where the sample is obtained non-invasively, such as a sample comprising buccal cells that is obtained using a buccal swab or brush.

The method described herein may be performed on nucleic acid extracts derived from the biological sample, in particular on genomic DNA. Genomic DNA may be obtained from DNA extracted directly from the biological sample or from cells obtained from such a sample. Methods of DNA extraction are well known in the art (see, for example, J. Sambrook et al., "Molecular Cloning: A Laboratory Manual", 2012, 4th Ed., Cold Spring Harbour Laboratory Press: New York). A classical DNA isolation protocol is based on extraction using organic solvents such as a mixture of phenol and chloroform, followed by precipitation with ethanol. Other methods include: salting out DNA extraction (see, for example, Sunnucks et al., Genetics, 1996, 144: 747-756; and Aljanabi et al., Nucl. Acids Res. 1997, 25: 4692-4693); the trimethylammonium bromide salts DNA extraction method (see, for example, Gustincich et al., BioTechniques, 1991, 11: 298-302) and the guanidinium thiocyanate DNA extraction method (see, for example, Hammond et al., Biochemistry, 1996, 240: 298-300).

There are also numerous different and versatile kits that can be used to extract DNA from biological tissues or fluids and that are commercially available for example from BD Biosciences Clontech (Palo Alto, CA), Epicentre Technologies (Madison, WI), Gentra Systems, Inc. (Minneapolis, MN), MicroProbe Corp. (Bothell, WA), Organon Teknika (Durham, NC), and Qiagen Inc. (Valencia, CA). Specific examples of such kits include, but are not limited to QIAamp^{RTM} Tissue Kit (Qiagen) and the Wizard^{RTM} Genomic DNA purification kit (Promega). User Guides that describe in great detail the protocol to be followed are usually included in all these kits. Sensitivity, processing time and cost may be different from one kit to another. One of ordinary skill in the art can easily select the kit(s) most appropriate for a particular situation.

### Methods of Determining the Identity of an Allele or of Obtaining a Genotype

A method described herein includes a step of determining the identity, presence or absence of alleles or genotypes associated with risk of developing canine glaucoma. Determination of the identity, presence or absence of alleles or genotypes associated with risk of developing canine glaucoma may be performed using any suitable method known in the art. Preferably, the method used is a SNP genotyping method. SNP genotyping methods include, but are not limited to, hybridization-based methods (such as dynamic allele-specific hybridization, molecular beacons, SNP microarrays), enzyme-based methods (such as restriction fragment length polymorphism (RFLP), PCR-based methods, FLAP endonuclease (such as the Invader assay), primer extension, 5'-nuclease, (such as the TaqMan™ assay), oligonucleotide ligation assays) and other post-amplification methods based on physical properties of DNA (such as single strand conformation polymorphism (SSCP), temperature gradient gel electrophoresis, denaturing high performance liquid chromatography, high resolution melting (HRM) of the entire amplicon, use of DNA mismatch-binding proteins, and SNPlex), and sequencing methods (such as direct manual sequencing, automated fluorescent sequencing and pyrosequencing).

In order to detect polymorphisms and/or polymorphic variants, it will frequently be desirable to amplify a portion of the target sequence (*e.g*., genomic DNA), for example a portion of the target sequence that encompasses the polymorphic site (SNP). The present method is not limited to any particular nucleic acid amplification technique, and any of a large variety of nucleic acid amplifications methods that are well-known in the art may be used in the practice of the method described herein (see, for example, Kimmel et al., Methods Enzymol. 1987, 152: 307-316; Sambrook et al., "Molecular Cloning: A Laboratory Manual", 2012, 4th Ed., Cold Spring Harbour Laboratory Press: New York, NY; *"*Short Protocols in Molecular Biology", Ausubel (Ed.), 2002, 5th Ed., John Wiley & Sons: Secaucus, NJ). Such nucleic acid amplification methods include, but are not limited to, the Polymerase Chain Reaction (or PCR, described in, for example, *"*PCR Protocols: A Guide to Methods and Applications", Innis (Ed.), 1990, Academic Press: New York; *"*PCR Strategies", Innis (Ed.), 1995, Academic Press: New York; *"*Polymerase chain reaction: basic principles and automation in PCR: A Practical Approach", McPherson et al. (Eds.), 1991, IRL Press: Oxford; Saiki et al., Nature, 1986, 324: 163; and U.S. Pat. Nos. 4,683,195, 4,683,202 and 4,889,818) and variations thereof including TaqMan™-based assays (Holland et al., Proc. Natl. Acad. Sci., 1991, 88: 7276-7280), and reverse transcriptase polymerase chain reaction (or RT-PCR, described in, for example, U.S. Pat. Nos. 5,322,770 and 5,310,652) and the like.

In PCR, a pair of primers is employed in excess to hybridize to the complementary strands of the target nucleic acid (*i.e.,* a portion of genomic DNA encompassing the SNP of interest). The primers are each extended by a DNA polymerase using the target sequence as a template. The extension products become targets themselves after dissociation (denaturation) from the original target strand. New primers are then hybridized and extended by the polymerase, and the cycle is repeated to exponentially increase the number of copies of amplicons. Examples of DNA polymerases capable of producing primer extension products in PCR reactions include, but are not limited to: *E. coli* DNA polymerase I, Klenow fragment of DNA polymerase I, T4 DNA polymerase, thermostable DNA polymerases isolated from *Thermus aquaticus* (Taq), available from a variety of sources (for example, Perkin Elmer), *Thermus thermophilus* (United States Biochemicals), *Bacillus stereothermophilus* (Bio-Rad), or *Thermococcus litoralis* ("Vent" polymerase, New England Biolabs). RNA target sequences may be amplified by reverse transcribing the mRNA into cDNA, and then performing PCR (RT-PCR). Alternatively, a single enzyme may be used for both steps as described in U.S. Pat. No. 5,322,770.

In addition to the enzymatic thermal amplification methods described above, isothermal enzymatic amplification reactions can be employed to amplify a portion of genomic DNA (gDNA) encompassing a SNP disclosed herein. These methods include, but are not limited to, Transcription-Mediated Amplification (or TMA, described in, for example, Kwoh et al., Proc. Natl. Acad. Sci. USA, 1989, 86: 1173-1177; Giachetti et al., J. Clin. Microbiol., 2002, 40: 2408-2419; and U.S. Pat. No. 5,399,491); Self-Sustained Sequence Replication (or 3SR, described in, for example, Guatelli et al., Proc. Natl. Acad. Sci. USA, 1990, 87: 1874-1848; and Fahy et al., PCR Methods and Applications, 1991, 1: 25-33); Nucleic Acid Sequence Based Amplification (or NASBA, described in, for example, Kievits et al., J. Virol., Methods, 1991, 35: 273-286; and U.S. Pat. No. 5,130,238) and Strand Displacement Amplification (or SDA, described in, for example, Walker et al., PNAS, 1992, 89: 392-396; EP 0 500 224 A2).

NASBA, 3SR and TMA primers require an RNA polymerase promoter linked to the target binding sequence of the primer. Promoters or promoter sequences for incorporation in the primers are nucleic acid sequences (either naturally occurring, produced synthetically or a product of a restriction digest) that are specifically recognized by an RNA polymerase that recognizes and binds to that sequence and initiates the process of transcription whereby RNA transcripts are generated. Examples of useful promoters include those which are recognized by certain bacteriophage polymerases such as those from bacteriophage T3, T7 or SP6 or a promoter from *E. Coli.*

Guidelines for selecting primers for PCR amplification are well known in the art. See, *e.g.,* McPherson et al., PCR Basics: From Background to Bench, Springer-Verlag, 2000. A variety of computer programs for designing primers are also available, *e.g.,* 'Oligo' (National Biosciences, Inc, Plymouth Minn.), MacVector (Kodak/IBI), and the GCG suite of sequence analysis programs (Genetics Computer Group, Madison, Wis. 53711).

An internal control or an internal standard may be added to the biological sample (or to purified nucleic acids extracted from the biological sample) to serve as a control for extraction and/or target amplification. The internal control generally includes a sequence that differs from the target sequence(s) and is capable of amplification by the primers used to amplify the target sequence comprising the SNP of interest. The use of an internal control allows the monitoring of the extraction process, amplification reaction, and detection, and control of the assay performance. The amplified control and amplified target are typically distinguished at the detection step by using different probes (*e.g.,* labeled with different detectable agents) for the detection of the control and the target.

A SNP described herein may be detected by sequencing the amplicons obtained by PCR. Any suitable method of sequencing may be used. For example, commercially available sequencing kits may be used, such as the BidDye® Terminator Sequencing Kit from Lifetechnology, the Sequenase™ Version 2.0 DNA Sequencing Kit from Affymetrix, the NEXTflex™ DNA Sequencing Kit from Bioo Scientific, and the like.

A SNP described herein may be detected using a primer extension assay. Briefly, an interrogation primer is hybridized to the sequence nucleotides immediately upstream of the SNP nucleotide. A DNA polymerase then extends the hybridized interrogation primer by adding a base that is complementary to the SNP. The primer sequence containing the incorporated base is then detected using methods known in the art. For example, the added base may be a fluorescently labelled nucleotide, or a hapten-labelled nucleotide recognized by antibodies.

Alternatively, a SNP described herein may be detected using restriction enzymes. For example, amplified products (amplicons) can be digested with a restriction enzyme that specifically recognizes a sequence comprising one of the SNP alleles, but does not recognize the other allele. PCR may be used to amplify DNA comprising a SNP, and amplified PCR products are subjected to restriction enzyme digestion under suitable conditions and restriction products are assessed. If, for example, a specific SNP allele corresponds to a sequence digested by the restriction enzyme, digestion is indicative of detection of that particular SNP allele. Restriction enzymes may be assayed electrophoretically, as is common in the art.

In some cases, dot-blot hybridization of amplified oligonucleotides with allele-specific oligonucleotide (ASO) probes can be performed.

Real-time quantitative PCR can also be used to determine copy number. Quantitative PCR permits both detection and quantification of specific DNA sequence in a sample as an absolute number of copies or as a relative amount when normalized to DNA input or other normalizing genes. A key feature of quantitative PCR is that the amplified DNA product is quantified in real-time as it accumulates in the reaction after each amplification cycle. Methods of quantification can include the use of fluorescent dyes that intercalate with double-stranded DNA, and modified DNA oligonucleotide probes that fluoresce when hybridized with a complementary DNA. Methods of quantification can include determining the intensity of fluorescence for fluorescently tagged molecular probes attached to a solid surface such as a microarray.

The method of detecting a SNP described herein comprises using a probe that specifically hybridizes to a nucleic acid region containing the SNP. For example, amplicons obtained by PCR of a biological sample obtained from a dog to be tested, are contacted with a composition comprising one or more probes under stringent hybridization conditions.

For example, the detection probes may be used in a TaqMan™ assay. A TaqMan™ assay, also known as fluorogenic 5' nuclease assay, is a powerful and versatile PCR-based detection system for nucleic acid targets. Analysis is performed in conjunction with thermal cycling by monitoring the generation of fluorescence signals. The assay system provides quantitative data allowing the determination of target copy numbers. The TaqMan™ assay is conveniently performed using, for example, AmpliTaq Gold™ DNA polymerase, which has endogenous 5' nuclease activity, to digest an oligonucleotide probe labeled with both a fluorescent reporter dye and a quencher moiety. Assay results are obtained by measuring changes in fluorescence that occur during the amplification cycle as the probe is digested, uncoupling the fluorescent and quencher moieties and causing an increase in the fluorescence signal that is proportional to the amplification of the target sequence.

Alternatively, probes may be used in heterogeneous methodologies. Heterogeneous detection systems are well-known in the art and generally employ a capture agent to separate amplified sequences from other materials in the reaction mixture. Capture agents typically comprise a solid support material (*e.g.*, microtiter wells, microarrays, beads, chips, and the like) coated with one or more specific binding sequences. A binding sequence may be complementary to a tail sequence added to oligonucleotide probes. Alternatively, a binding sequence may be complementary to a sequence of a capture oligonucleotide, itself comprising a sequence complementary to a tail sequence of an oligonucleotide probe. After separation of the amplification product/probe hybrids bound to the capture agents from the remaining reaction mixture, the amplification product/probe hybrids can be detected using any suitable detection methods.

For example, in the DNA array method, DNA samples are amplified by PCR, and the amplified products are labeled. For labeling amplified products, tagged primers are used. For example, genomic DNA is first amplified by PCR with a primer set that is specific for a region comprising the polymorphic site of interest. Subsequently, labeling PCR is performed using biotin-labeled primers to thereby synthesize biotin-labeled DNA. The thus synthesized biotin-labeled DNA is allowed to hybridize to oligonucleotide probes on the chip. The reaction mixture and conditions for hybridization may be adjusted appropriately depending on the length of the nucleotide probes to be immobilized to the solid phase and conditions such as reaction temperature. One of ordinary skill in the art would be able to design appropriate hybridization conditions. For detecting hybridized DNA, avidin labeled with a fluorescent dye is added. The DNA array is analyzed with a scanner and checked for the presence or absence of hybridization using fluorescence as an indicator.

A method described herein can include determining the genotype of a subject with respect to both copies of the polymorphic site present in the genome. For example, the complete genotype may be characterized as -/-, as -/+, or as +/+, where a minus sign indicates the presence of the reference or wild type sequence at the polymorphic site, and the plus sign indicates the presence of a polymorphic variant other than the reference sequence. If multiple polymorphic variants exist at a site, this can be appropriately indicated by specifying which ones are present in the subject. Any of the detection means described herein can be used to determine the genotype of a subject with respect to one or both copies of the polymorphism present in the subject's genome.

### Identification of Risk of Developing Glaucoma

In a method described herein, the dog tested is identified as being at risk of having or developing canine glaucoma if the variant allele of rs9172407 is T at nucleotide position 401 of the flanking sequence SEQ ID NO: 1 and/or if the variant allele of rs22115601 is T at nucleotide position 401 of the flanking sequence SEQ ID NO: 2. Alternatively, the dog tested is identified as being at risk of having or developing canine glaucoma if the genotype C/T or the genotype T/T is identified for SNP rs9172407 and/or if the genotype C/T or the genotype T/T is identified for SNP rs22115601.

Once a dog tested has been identified as being at risk of having or developing canine glaucoma using a method described herein, a veterinary professional may (optionally after having performed a complete eye examination) select and prescribe an appropriate and customized treatment for the dog depending on the advancement of the disease. The goal of treating early forms of glaucoma is to maintain a normal intraocular pressure and to preserve vision. Common medications for early glaucoma include medications to decrease aqueous humor production such as β-blockers (*e.g.,* betaxolol, and timolol maleate), and carbonic anhydrase inhibitors (*e.g.,* methazolamide, brinzolamide, dorzolamide, and dorzolamide-timolol maleate); and medications to increase aqueous humor outflow such as cholinergics (*e.g.,* pilocarpine, and demecarium bromide), prostaglandin analogs (*e.g.,* latanoprost, travoprost, and bimatoprost); and hyperosmotics (*e.g.,* mannitol and glycerin). The medical treatment prescribed may include a combination of medications. If necessary, different surgical options exist to attempt to permanently normalize the intraocular pressure. Such options include, for example, aqueous humor shunts, cyclodestructive procedures, enucleation, intrascleral prostheses, and chemical ablation.

Once a dog tested has been identified as being at risk of developing glaucoma using a method described herein, a veterinary professional may alternatively recommend increasing the frequency of veterinary eye examinations for the dog and/or may prescribe a treatment for delaying the onset of the disease. For example, the onset of glaucoma may be delayed by providing the dog with a daily canine antioxidant vision supplement, such as OcuGLO™.

Furthermore, the result of a method described herein may help dog owners make better breeding decisions. Indeed, carefully selecting prospective mating partners (*i.e.,* dogs that do not exhibit the glaucoma risk allele(s) or genotype) may hopefully reduce the incidence of primary glaucoma.

### III - Primers and Probes for SNP Allele Identification and/or SNP Genotyping

Also described herein is a reagent for use in *an vitro* method described herein. The reagent may be any molecule that allows the determination of the identity, presence and/or absence of alleles or genotypes associated with risk of developing canine glaucoma, and more particularly to the identity, presence and/or absence of alleles or genotypes of at least one SNP described herein, *i.e.,* SNP rs9172407 and SNP rs22115601. Preferably, the reagent is selected from the group consisting of amplification primers, detection probes, and any combination thereof.

The primers and probes described herein may be oligonucleotides comprising at least 15 nucleotides.

When the oligonucleotide is used as a primer, its length is usually 15 to 100 nucleotides, preferably 17 to 30 nucleotides. The length of DNA that primers can amplify (*i.e.,* the length of the target sequence comprising the SNP of interest) is usually 15-1000 nucleotides, preferably 20-500 nucleotides, more preferably 20-200 nucleotides. The primer is not particularly limited as long as it is capable of amplifying at least a part of a DNA comprising a SNP described herein. In the case of SNP rs9172407, the target sequence to be amplified is a region of SEQ ID NO: 1 that comprises the nucleotide position 401 in SEQ ID NO: 1. In the case of SNP rs22115601, the target sequence to be amplified is a region of SEQ ID NO: 2 that comprises the nucleotide position 401 in SEQ ID NO: 2. From the sequences set forth in SEQ ID NO: 1 and SEQ ID NO: 2, one skilled in the art knows how to design and prepare suitable primers.

A reagent described herein may be a pair of primers comprising: a 5' (upstream) primer (or forward primer) that hybridizes with the 5' end of the nucleic acid sequence to be amplified and a 3' (downstream) primer (or reverse primer) that hybridizes with the complement of the sequence to be amplified. Such primer pairs are particularly useful in PCR amplification reactions.

For example, a primer pair capable of amplifying a nucleic acid target comprising SNP rs9172407 may comprise:
- a forward primer comprising, or consisting of, the sequence set forth in SEQ ID NO: 3 (5'-CTTCCTTGCTTCCCCTCTTG-3'); and
- a reverse primer comprising, or consisting of, the sequence set forth in SEQ ID NO: 4 (5'-GAACCTGAAATGGCAAAGGA-3').

This primer pair amplifies a region of SEQ ID NO: 1 that is 286 nucleotide-long and that comprises the nucleotide position 401 in SEQ ID NO: 1.

For example, a primer pair capable of amplifying a nucleic acid target comprising SNP rs22115601 may comprise:
- a forward primer comprising, or consisting of, the sequence set forth in SEQ ID NO: 5 (5'-CAAATGCCAATAAGCACATGA-3'); and
- a reverse primer comprising, or consisting of, the sequence set forth in SEQ ID NO: 6 (5'-GGGGACAAGTCAATGGTTTC-3').

This primer pair amplifies a region of SEQ ID NO: 2 that is 240 nucleotide-long and that comprises the nucleotide position 401 in SEQ ID NO: 2.

In addition to a nucleotide sequence identical or complementary to the nucleotide sequence of the target sequence, the primer may comprise any nucleotide sequence added thereto (as described above). For example, in primers intended to be used in polymorphism analysis with IIs type restriction enzyme, a recognition sequence for IIs type restriction enzyme is added to the primer. Furthermore, the primer may be modified. For example, primers may be labeled with a detectable agent or moiety (*e.g.,* a fluorescent substance or a binding affinity substance such as biotin or digoxin).

These primer pairs can be used according to any nucleic acid amplification technique that employs two or more oligonucleotides to amplify a target sequence (as discussed above). Amplification products produced using primer pair described herein may be detected using any of a variety of detection methods well known in the art. For example, amplification products may be detected using agarose gel electrophoresis and visualization by ethidium bromide staining and exposure to ultraviolet (UV) light or by sequence analysis of the amplification product for confirmation of the SNP allele, or any other method known in the art.

When the oligonucleotide is used as a probe, its length is usually 5 to 200 nucleotides, preferably 7 to 100 nucleotides, more preferably 7 to 50 nucleotides. The probe is not particularly limited as long as it is capable of specifically hybridizing to a DNA comprising a SNP described herein. In the case of SNP rs9172407, the target sequence to which the probe is capable of specifically hybridizing is a region of the flanking sequence SEQ ID NO: 1 that comprises the nucleotide position 401 in the flanking sequence SEQ ID NO: 1. In the case of SNP rs22115601, the target sequence to which the probe is capable of specifically hybridizing is a region of the flanking sequence SEQ ID NO: 2 that comprises the nucleotide position 401 in the flanking sequence SEQ ID NO: 2. From the sequences set forth in SEQ ID NO: 1 and SEQ ID NO: 2, one skilled in the art knows how to the design and prepare suitable probes.

Generally probes have a complimentary nucleic acid sequence that selectively hybridizes to the target nucleic acid sequence. In order for a probe to hybridize to a target sequence, the hybridization probe must have sufficient identity with the target sequence, *i.e.,* at least 70% (e.g., 80%, 90%, 95%, 98% or more) identity to the target sequence. The probe sequence must also be sufficiently long so that the probe exhibits selectivity for the target sequence over non-target sequences. Probes include primers, which generally refers to a single-stranded oligonucleotide probe that can act as a point of initiation of template-directed DNA synthesis using methods such as PCR (polymerase chain reaction), LCR (ligase chain reaction), etc., for amplification of a target sequence.

Preferred is a probe which specifically hybridizes to a DNA having the nucleotide sequence of the region comprising a SNP described herein. Alternatively, depending on the method of analysis of the nucleotide at the polymorphic site, a probe may be designed so that its end corresponds to nucleotides adjacent to the polymorphic site. Therefore, a probe which does not comprise the polymorphic site in its nucleotide sequence but comprises a nucleotide sequence complementary to the region adjacent to the polymorphic site therein is also a probe described herein.

Like the primers, the probes described herein can exhibit an alteration of their nucleotide sequence, an addition of a nucleotide sequence, or any other modification. For example, a nucleotide sequence that constitutes FLAP and which is foreign to the genome is added to probes for use in the Invader method. Such probes are also included in the probes described herein as long as they hybridize to a region comprising a SNP disclosed herein. The nucleotide sequence constituting the probe described herein may be designed based on the nucleotide sequences of peripheral DNA regions around the polymorphic site described herein in the genome, depending on the analysis method to be employed.

A peptide nucleic acid (PNA) probe can be used instead of a nucleic acid probe in the hybridization methods described above. PNA is a DNA mimetic with a peptide-like, inorganic backbone, *e.g.,* N-(2-aminoethyl)glycine units, with an organic base (A, G, C, T or U) attached to the glycine nitrogen via a methylene carbonyl linker (see, e.g., Nielsen et al., Bioconjugate Chemistry, The American Chemical Society, 1994, 5: 1). A PNA probe can be designed to specifically hybridize to a nucleic acid comprising a polymorphic variant.

Thus, it is to be understood that an oligonucleotide described herein (primer or probe) may include one or more sequences which can serve as spacers, linkers, sequences for labeling or binding to an enzyme, which may impart added stability or susceptibility to degradation process or other desirable property to the oligonucleotide.

Also described herein are primers and probes designed using the nucleotide sequence complementary to sequence SEQ ID NO: 1 to determine the identity, presence or absence of SNP rs9172407, as well as primers and probes designed using the nucleotide sequence complementary to sequence SEQ ID NO: 2 to determine the identity, presence or absence of rs22115601.

### Preparation of Primers and Probes

The primers and probes described herein may be prepared using any of a variety of methods well known in the art (see, for example, J. Sambrook et al., "Molecular Cloning: A Laboratory Manual", 2012, 4th Ed., Cold Spring Harbour Laboratory Press: New York, NY; *"*PCR Protocols: A Guide to Methods and Applications", 1990, M.A. Innis (Ed.), Academic Press: New York, NY; P. Tijssen "Hybridization with Nucleic Acid Probes - Laboratory Techniques in Biochemistry and Molecular Biology (Parts I and II)", 1993, Elsevier Science; *"*PCR Strategies", 1995, M.A. Innis (Ed.), Academic Press: New York, NY; and *"*Short Protocols in Molecular Biology", 2002, F.M. Ausubel (Ed.), 5th Ed., John Wiley & Sons: Secaucus, NJ). For example, oligonucleotides may be prepared by chemical synthesis and polymerization based on a template as described, *e.g.,* in S.A. Narang et al., Meth. Enzymol. 1979, 68: 90-98; E.L. Brown et al., Meth. Enzymol. 1979, 68: 109-151; E.S. Belousov et al., Nucleic Acids Res. 1997, 25: 3440-3444; D. Guschin et al., Anal. Biochem. 1997, 250: 203-211; M.J. Blommers et al., Biochemistry, 1994, 33: 7886-7896; and K. Frenkel et al., Free Radic. Biol. Med. 1995, 19: 373-380; and U.S. Pat. No. 4,458,066).

For example, oligonucleotides may be prepared using an automated, solid-phase procedure based on the phosphoramidite approach. In such a method, each nucleotide is individually added to the 5'-end of a growing oligonucleotide chain, which is attached at the 3'-end to a solid support. The added nucleotides are in the form of trivalent 3'-phosphoramidites that are protected by a dimethoxythriyl (or DMT) group at the 5' position. After based-induced phosphoramidite coupling, mild oxidation to give a pentavalent phosphotriester intermediate and DMT removal provides a new site for oligonucleotide elongation. The oligonucleotides are then cleaved off the solid support, and the phosphodiester and exocyclic amino groups are deprotected with ammonium hydroxide. These syntheses may be performed on oligo synthesizers such as those commercially available from Perkin Elmer/Applied Biosystems, Inc. (Foster City, CA), DuPont (Wilmington, DE) or Milligen (Bedford, MA). Alternatively, oligonucleotides can be custom made and ordered from a variety of commercial sources well-known in the art, including, for example, the Midland Certified Reagent Company (Midland, TX), ExpressGen, Inc. (Chicago, IL), Operon Technologies, Inc. (Huntsville, AL), BioSearch Technologies, Inc. (Novato, CA), and many others.

Purification of oligonucleotides described herein, where necessary or desired, may be carried out by any of a variety of methods well known in the art. Purification of oligonucleotides is typically performed either by native acrylamide gel electrophoresis, by anion-exchange HPLC as described, for example, by Pearson et al. (J. Chrom., 1983, 255: 137-149) or by reverse phase HPLC (McFarland et al., Nucleic Acids Res., 1979, 7: 1067-1080).

The sequence of oligonucleotides can be verified using any suitable sequencing method including, but not limited to, chemical degradation (Maxam et al., Methods of Enzymology, 1980, 65: 499-560), matrix-assisted laser desorption ionization time-of-flight (MALDI-TOF) mass spectrometry (Pieles et al., Nucleic Acids Res., 1993, 21: 3191-3196), mass spectrometry following a combination of alkaline phosphatase and exonuclease digestions (Wu et al., Anal. Biochem., 2001, 290: 347-352), and the like.

As already mentioned above, modified oligonucleotides may be prepared, from the oligonucleotide sequences provided herein (see SEQ ID NOs: 3-6), using any of several means known in the art. Non-limiting examples of such modifications include methylation, "caps", substitution of one or more of the naturally occurring nucleotides with an analog, and internucleotide modifications such as, for example, those with uncharged linkages (*e.g.,* methyl phosphonates, phosphotriesters, phosphoroamidates, carbamates, etc), or charged linkages (*e.g.,* phosphorothioates, phosphorodithioates, etc). Oligonucleotides may contain one or more additional covalently linked moieties, such as, for example, proteins (*e.g.,* nucleases, toxins, antibodies, signal peptides, poly-L-lysine, etc), intercalators (*e.g.,* acridine, psoralen, etc), chelators (*e.g.,* to chelate metals, radioactive metals, oxidative metals, etc), and alkylators. Oligonucleotides may also be derivatized by formation of a methyl or ethyl phosphotriester or an alkyl phosphoramidate linkage. Furthermore, the oligonucleotide sequences described herein may also be modified with a label.

### Labeling of Primers and/or Probes

Detection probes or amplification primers or both probes and primers are labeled with a detectable agent or moiety before being used in amplification/detection assays. Preferably, the detection probes are labeled with a detectable agent. The role of a detectable agent is to allow visualization and detection of amplified target sequences (amplicons). Preferably, the detectable agent is selected such that it generates a signal which can be measured and whose intensity is related (*e.g.,* proportional) to the amount of amplification products in the sample being analyzed.

The association between an oligonucleotide (*e.g.,* detection probe) and a detectable agent can be covalent or non-covalent. Labeled detection probes can be prepared by incorporation of, or conjugation to, a detectable moiety. Labels can be attached directly to the nucleic acid sequence or indirectly (*e.g.,* through a linker). Linkers and spacer arms of various lengths are known in the art and are commercially available, and can be selected to reduce steric hindrance, or to confer other useful or desired properties to the resulting labeled molecules (see, for example, Mansfield et al., Mol. Cell Probes, 1995, 9: 145-156).

Methods for labeling nucleic acid molecules are well-known in the art. For a review of labeling protocols, label detection techniques, and developments in the field, see, for example, Kricka, Ann. Clin. Biochem. 2002, 39: 114-129; van Gijlswijk et al., Expert Rev. Mol. Diagn. 2001, 1: 81-91; and Joos et al., J. Biotechnol. 1994, 35: 135-153. Standard nucleic acid labeling methods include: incorporation of radioactive agents, direct attachments of fluorescent dyes or of enzymes; chemical modifications of nucleic acid molecules making them detectable immunochemically or by other affinity reactions; and enzyme-mediated labeling methods, such as random priming, nick translation, PCR and tailing with terminal transferase (for a review on enzymatic labeling, see, for example, Temsamani et al., Mol. Biotechnol. 1996, 5: 223-232). Other nucleic acid labeling systems include, but are not limited to: ULS (Universal Linkage System), which is based on the reaction of monoreactive cisplatin derivatives with the N7 position of guanine moieties in DNA (Heetebrij et al., Cytogenet. Cell. Genet. 1999, 87: 47-52), psoralen-biotin, which intercalates into nucleic acids and upon UV irradiation becomes covalently bonded to the nucleotide bases (Levenson et al., Methods Enzymol. 1990, 184: 577-583; and Pfannschmidt et al., Nucleic Acids Res. 1996, 24: 1702-1709), photoreactive azido derivatives (Neves et al., Bioconjugate Chem. 2000, 11: 51-55), and DNA alkylating agents (Sebestyen et al., Nat. Biotechnol. 1998, 16: 568-576).

Any of a wide variety of detectable agents can be used for labeling an oligonucleotide described herein. Suitable detectable agents include, but are not limited to, various ligands, radionuclides (such as, for example, ³²P, ³⁵S, ³H, ¹⁴C, ¹²⁵I, ¹³¹I, and the like); fluorescent dyes (see below); chemiluminescent agents (such as, for example, acridinium esters, stabilized dioxetanes, and the like); spectrally resolvable inorganic fluorescent semiconductor nanocrystals (*i.e.,* quantum dots), metal nanoparticles (*e.g.,* gold, silver, copper and platinum) or nanoclusters; enzymes (such as, for example, those used in an ELISA, *i.e.,* horseradish peroxidase, beta-galactosidase, luciferase, alkaline phosphatase); colorimetric labels (such as, for example, dyes, colloidal gold, and the like); magnetic labels (such as, for example, Dynabeads™); and biotin, dioxigenin or other haptens and proteins for antisera or monoclonal antibodies are available.

Preferably, detection probes are fluorescently labeled. Numerous known fluorescent labeling moieties of a wide variety of chemical structures and physical characteristics are suitable for labelling an oligonucleotide described herein. Suitable fluorescent dyes include, but are not limited to, fluorescein and fluorescein dyes (*e.g.,* fluorescein isothiocyanine or FITC, naphthofluorescein, 4',5'-dichloro-2',7'-dimethoxy-fluorescein, 6-carboxyfluorescein or FAM), carbocyanine, merocyanine, styryl dyes, oxonol dyes, phycoerythrin, erythrosin, eosin, rhodamine dyes (*e.g.,* carboxytetramethylrhodamine or TAMRA, carboxyrhodamine 6G, carboxy-X-rhodamine (ROX), lissamine rhodamine B, rhodamine 6G, rhodamine Green, rhodamine Red, tetramethylrhodamine or TMR), coumarin and coumarin dyes (*e.g.,* methoxycoumarin, dialkylaminocoumarin, hydroxycoumarin and amino-methylcoumarin or AMCA), Oregon Green Dyes (*e.g.,* Oregon Green 488, Oregon Green 500, Oregon Green 514), Texas Red, Texas Red-X, Spectrum Red™, Spectrum Green™, cyanine dyes (*e.g.,* Cy-3™, Cy-5™, Cy-3.5™, Cy-5.5™), Alexa Fluor dyes (*e.g.,* Alexa Fluor 350, Alexa Fluor 488, Alexa Fluor 532, Alexa Fluor 546, Alexa Fluor 568, Alexa Fluor 594, Alexa Fluor 633, Alexa Fluor 660 and Alexa Fluor 680), BODIPY dyes (*e.g.,* BODIPY FL, BODIPY R6G, BODIPY TMR, BODIPY TR, BODIPY 530/550, BODIPY 558/568, BODIPY 564/570, BODIPY 576/589, BODIPY 581/591, BODIPY 630/650, BODIPY 650/665), IRDyes (*e.g.,* IRD40, IRD 700, IRD 800), and the like. For more examples of suitable fluorescent dyes and methods for linking or incorporating fluorescent dyes to nucleic acid molecules see, for example, *"*The Handbook of Fluorescent Probes and Research Products", 9th Ed., Molecular Probes, Inc., Eugene, OR. Fluorescent dyes as well as labeling kits are commercially available from, for example, Amersham Biosciences, Inc. (Piscataway, NJ), Molecular Probes Inc. (Eugene, OR), and New England Biolabs Inc. (Berverly, MA).

Rather than being directly detectable themselves, some fluorescent groups (donors) transfer energy to another fluorescent group (acceptor) in a process of fluorescent resonance energy transfer (FRET), and the second group produces the detectable fluorescent signal. In such cases, the oligonucleotide detection probe may, for example, become detectable when hybridized to an amplified target sequence. Examples of FRET acceptor/donor pairs suitable for use to label detection probes described herein include, but are not limited to, fluorescein/tetramethylrhodamine, IAEDANS/FITC, IAEDANS/5-(iodoacetomido)fluorescein, B-phycoerythrin/Cy-5, FAM/TAMRA and EDANS/Dabcyl.

The use of physically linked fluorescent reporter/quencher molecule pairs is also possible. The use of such systems in TaqMan™ assays (as described, for example, in U.S. Pat. Nos. 5,210,015; 5,804,375; 5487,792 and 6214,979) or as Molecular Beacons (as described, for example in, Tyagi et al., Nature Biotechnol. 1996, 14: 303-308; Tyagi et al., Nature Biotechnol. 1998, 16: 49-53; Kostrikis et al., Science, 1998, 279: 1228-1229; Sokol et al., Proc. Natl. Acad. Sci. USA, 1998, 95: 11538-11543; Marras et al., Genet. Anal. 1999, 14: 151-156; and U.S. Pat. Nos. 5,846,726, 5,925,517, 6,277,581 and 6,235,504) is well-known in the art. With the TaqMan™ assay format, products of the amplification reaction can be detected as they are formed in a so-called "real-time" manner. As a result, amplification product/probe hybrids are formed and detected while the reaction mixture is under amplification conditions.

The PCR detection probes may be TaqMan™-like probes that are labeled at the 5'-end with a fluorescent moiety and at the 3'-end with a quencher moiety. Suitable fluorophores and quenchers for use with TaqMan™-like probes are disclosed in U.S. Pat. Nos. 5,210,015; 5,804,375; 5,487,792; and 6,214,979; and WO 01/86001. Examples of quenchers include, but are not limited to DABCYL (*i.e.,* 4-(4'-dimethylaminophenylazo)-benzoic acid) succinimidyl ester, diarylrhodamine carboxylic acid, succinimidyl ester (or QSY-7), and 4',5'-dinitrofluorescein carboxylic acid, succinimidyl ester (or QSY-33) (all available, for example, from Molecular Probes), quencher1 (Q1; available from Epoch Biosciences, Bothell, WA), or "Black hole quenchers" BHQ-1, BHQ-2, and BHQ-3 (available from BioSearch Technologies, Inc., Novato, CA).

A "tail" of normal or modified nucleotides can also be added to oligonucleotide probes for detectability purposes. A second hybridization with a nucleic acid molecule complementary to the tail and containing one or more detectable labels allows visualization of the amplicon/probe hybrids (see, for example, the system commercially available from Enzo Biochem, Inc. New York, NY). Another example of an assay with which the oligonucleotides described herein are useful is a signal amplification method such as that described in U.S. Pat. No. 5,124,246. In that method, the signal is amplified through the use of amplification multimers, polynucleotides which are constructed so as to contain a first segment that hybridizes specifically to the "tail" added to the oligonucleotide probes, and a multiplicity of identical second segments that hybridize specifically to a labeled probe. The degree of amplification is theoretically proportional to the number of iterations of the second segment. The multimers may be either linear or branched. Branched multimers may be in the shape of a fork or a comb.

The selection of a particular nucleic acid labeling technique will depend on the situation and will be governed by several factors, such as the ease and cost of the labeling method, the quality of sample labeling desired, the effects of the detectable moiety on the hybridization reaction (*e.g.,* on the rate and/or efficiency of the hybridization process), the nature of the amplification method used, the nature of the detection system, the nature and intensity of the signal generated by the detectable label, and the like.

### Immobilization of Probes

A detection probe may be immobilized on a solid support. For example, the solid support may be a bead, a particle, a microplate well, an array, a cuvette, a tube, a membrane, a gel, a resin, and the like.

A probe may be immobilized by being either covalently or non-covalently bound to the surface of a solid carrier or support. The term "solid support" refers to a material to which nucleic acid molecules can be immobilized. The solid support is not particularly limited as long as it is capable of immobilizing nucleic acid molecules. Thus, the solid support may be any solid support known in the art to which the probe can be operably affixed. "Operably affixed" refers to the probe being affixed in a manner permitting the formation of a complex between the affixed probe and the amplicons (or the target sequence) present in the biological sample tested. Examples of suitable carrier or support materials include, but are not limited to, agarose, cellulose, nitrocellulose, dextran, Sephadex, Sepharose, carboxymethyl cellulose, polyacrylamides, polystyrene, polyvinyl chloride, polypropylene, gabbros, magnetic ion-exchange resin, glass, polyamine-methyl-vinyl-ether-maleic acid copolymer, amino acid copolymer, ethylene-maleic acid copolymer, nylon, silk, and the like. The solid carrier or support may be in the form of a bead, a particle, a microplate well, an array, a cuvette, a tube, a membrane or any other shape suitable for SNP analysis. Immobilization of a probe to a solid carrier or support may include gel electrophoresis followed by transfer to a membrane (typically nitrocellulose or PVDF) in a process called western blotting (or immunoblot) well known in the art.

Methods for generating arrays are known in the art and include, *e.g.,* photolithographic methods (see, *e.g.,* U.S. Pat. Nos. 5,143,854; 5,510,270; and 5,527,681), mechanical methods (*e.g.,* directed-flow methods as described in U.S. Pat. No. 5,384,261), pin-based methods (*e.g.,* as described in U.S. Pat. No. 5,288,514), and bead-based techniques (*e.g.,* as described in PCT US/93/04145). The array typically includes oligonucleotide hybridization probes capable of specifically hybridizing to different polymorphic variants. Oligonucleotide probes that exhibit differential or selective binding to polymorphic sites may readily be designed by one of ordinary skill in the art. For example, an oligonucleotide that is perfectly complementary to a sequence that encompasses a polymorphic site (*i.e.,* a sequence that includes the polymorphic site, within it or at one end) will generally hybridize preferentially to a nucleic acid comprising that sequence, as opposed to a nucleic acid comprising an alternate polymorphic variant.

Oligonucleotide probes forming an array may be attached to a substrate by any number of techniques, including, without limitation, (i) *in situ* synthesis (*e.g.,* high-density oligonucleotide arrays) using photolithographic techniques; (ii) spotting/printing at medium to low density on glass, nylon or nitrocellulose; (iii) by masking, and (iv) by dot-blotting on a nylon or nitrocellulose hybridization membrane. Oligonucleotides can be immobilized *via* a linker, including by covalent, ionic, or physical linkage. Linkers for immobilizing nucleic acids and polypeptides, including reversible or cleavable linkers, are known in the art. See, for example, U.S. Pat. No. 5,451,683 and WO98/20019.

### IV - Kits for Canine Glaucoma Testing

Also described herein are kits comprising material useful for canine glaucoma testing according to a method disclosed above. In particular, kits for identifying dogs at risk of having or developing glaucoma are described herein, that contain material allowing the detection, in a DNA sample of a dog to be tested, of at least one of the SNPs disclosed herein. Using such a kit, a canine glaucoma testing method may be performed by diagnostic laboratories, by veterinary physicians, or by pet owners.

Materials and reagents for testing biological samples from dogs may be assembled together in a kit. A kit can be designed so as to be used with a particular amplification technique, such as for example a PCR technique. Each kit preferably comprises the reagents which render the procedure specific. Thus, a kit intended to be used with NASBA preferably contains primers with a RNA polymerase promoter linked to the target binding sequence, while a kit intended to be used with SDA preferably contains primers including a restriction endonuclease recognition site 5' to the target binding sequence. Similarly, when the kit is intended to be used in a 5' nuclease assay, such as the TaqMan™ assay, the detection probes preferably contain at least one fluorescent reporter moiety and at least one quencher moiety.

In general, a kit comprises a pair of primers described herein allowing the amplification of a DNA region comprising at least one SNP disclosed herein. Thus, a kit may comprise a set of primers allowing the amplification of a region of the dog's genomic DNA that contains SNP rs9172407 and/or a set of primers allowing the amplification of a region of the dog's genomic DNA that contains SNP rs22115601, as described above. Additionally or alternatively, a kit may comprise at least one probe capable of specifically hybridizing a region of the dog's genomic DNA that contains SNP rs9172407 and/or at least one probe capable of specifically hybridizing a region of the dog's genomic DNA that contains SNP rs9172407, as described above. The probe(s) may be immobilized on a solid support.

The kit may comprise amplification reaction reagents. Suitable amplification reaction reagents include, for example, one or more of: buffers, reagents, enzymes having reverse transcriptase and/or polymerase activity or exonuclease activity, enzyme cofactors such as magnesium or manganese; salts; deocynucleotide triphosphates (such as dATP, dGTP, dCTP, dTTP, and dUTP) suitable for carrying out the amplification reaction. For example, a kit, intended to be used with NASBA, may contain suitable amounts of reverse transcriptase, RNase H and T7 RNA polymerase. In kits intended to be used in transcription amplification reactions, such as NASBA, buffers can be included that contain, for example, DMSO, which is known to enhance the amplification reaction.

The kit may comprise an internal control as a check on the amplification procedure, and to prevent occurrence of false negative test results due to failures in the amplification procedure. An optimal control sequence is selected in such a way that it will not compete with the target nucleic acid sequence in the amplification reaction (as described above).

Depending on the procedure, the kit may further comprise one or more of: extraction buffer and/or reagents (in particular genomic DNA extraction), wash buffers and/or reagents, hybridization buffer and/or reagents, labeling buffer and/or reagents (*e.g.,* one or more of the same or different labels for the probes), and detection means. Protocols for using these buffers and reagents to perform different steps of the procedure may be included in the kit.

The reagents may be supplied in a solid (*e.g.,* lyophilized) or liquid form. The kits may include media for the reconstitution of lyophilized ingredients. The kits may optionally comprise different containers (*e.g.,* vial, ampoule, test tube, flask or bottle) for each individual buffer and/or reagent. Each component will generally be suitable as aliquoted in its respective container or provided in a concentrated form. Other containers suitable for conducting certain steps of the disclosed methods may also be provided. The individual containers of the kit are preferably maintained in close confinement for commercial sale.

A kit may further comprise instructions for using the kit according to a method described herein. Instructions for using the kit according to such a method may comprise instructions for processing the biological sample obtained from the dog to be tested, and/or instructions for amplifying a region of the dog's genomic DNA that contains a SNP disclosed herein, and/or instructions for labelling primers, and/or probes, and/or instructions for performing the test, and/or instructions for interpreting the results as well as a notice in the form prescribed by a governmental agency (*e.g.,* FDA) regulating the manufacture, use or sale of pharmaceuticals or biological products.

The kit may comprise a means for collecting the biological sample. For example, the kit may comprise one or more buccal swabs or brushes.

An identifier, *e.g.,* a bar code, radio frequency, ID tags, etc., may be present in or on the kit. The identifier can be used, for example, to uniquely identify the kit for purposes of quality control, inventory control, tracking movement between workstations, etc.

Unless otherwise defined, all the technical and scientific terms used herein have the same meaning as generally understood in the field. Further aspects and advantages of this invention will be disclosed in the following examples.

### Examples

The following examples describe some of the preferred modes of making and practicing the present invention. However, it should be understood that the examples are for illustrative purposes only and are not meant to limit the scope of the invention.

### Materials and Methods

### Dogs and Dog populations

For the across breed studied, blood samples from a population of 77 healthy dogs and from a population of 135 glaucomatous dogs, characterized as shown below, were used in the present Examples. This study was conducted in collaboration with the Centre Hospitalier Vétérinaire (Bellevue, France).

The dogs of the two populations were 59% males and their average age was: 7.2 years. In the population of healthy dogs, the average IOP was such that OD (IOP in the right eye) = 14.81 and OS (IOP in the left eye) = 15.12. In the population of glaucomatous dogs, the average IOP was such that OD = 49.98 (53% of seeing eyes) and OS = 50.87 (50% of seeing eyes). The glaucoma diagnosis was performed using tonometry (TonoVet Tonometer from Icare Finland Oy, Finland), biomicroscopy (instrument KOWA SL 15, from KOWA, USA), biometry (instrument OPKO OTI from Optos, USA) and electrophysiology (ERG, Pupillscan, SIEM Biomédicale, France).

The dogs from the populations studied were from the following breeds: French Bulldog, Boxer, Bichon, German Shepherd, Short-Haired Dachshund, Golden Retriever, Border Collie, Newfoundland, Griffon Vendéen, Australian Shepherd, Long-Haired Dachshund, Groenendael (or Belgian Sheepdog), Doberman, Beauceron, Weimaraner, Bull Terrier, Hovawart, Polish Lowland Sheepdog, Labrador, Great Dane, Jack Russell Terrier, Shar Pei, Pointer, English Setter, Poodle, Shi-Tzu, Jack Terrier, Husky, Gordon Setter, Fox Terrier, Korthals Griffon, American Cocker, Cocker, Jagdterrier, Brittany, Italian Greyhound, Beagle, Labrador, Pomeranian, Lhassa apso, Cavalier King Charles Spaniel, Tervuren, Bouvier des Flandres, Staffordshire Bull Terrier, Flat-Coated Retriever, West Highland White Terrier (or Westie), Irish Setter, Schnauzer, English Setter, Basset Hound, York, Pinscher, Leonberger, Husky, and Eurasier.

For the within breed studied, blood samples from a population of 13 healthy Golden Retrievers and from a population of 8 glaucomatous Golden Retrievers, and blood samples from a population of 13 healthy French Bulldogs and from a population of 13 glaucomatous French Bulldogs were used. The Golden Retrievers and French Bulldogs were from the populations described above.

In addition, blood samples from a population of 4 healthy Eurasiers and from a population of 26 glaucomatous Eurasiers from the European Network in Veterinary Ophthalmology & Animal Vision (http://www.reovva.com/) were used.

### SNPs and Primer Pairs

In the *SRBD1* gene, a total of 4 polymorphisms were selected: rs22018514, rs22018513, rs9172407, and rs22115601, with rs22018514 and rs22018513 having been previously described (see Kanemaki et al., PLoS one, 2013, 8(9): e74372 and US 2014/0141432). In the *CYP1B1* gene, which has previously been described as a candidate gene in Beagles with primary open-angle glaucoma (Kato et al., Molecular Vision, 2009, 15: 2470), a total 3 polymorphisms were selected (rs22594186, rs22594187, rs22594188).

The primer pairs that were used in the examples below are presented in Table 1.

**Table 1. Primer pairs used in the PCR reactions (wherein F = forward primer and R = reverse primer).**

| **SNPs** | **Primers Sequences** | **SEQ ID NOs** | **Amplicon Size (bp)** |
|---|---|---|---|
| ***SRBD1* gene** | | | |
| **rs9172407** | F: CTTCCTTGCTTCCCCTCTTG | SEQ ID NO: 3 | 286 |
| | R: GAACCTGAAATGGCAAAGGA | SEQ ID NO: 4 | |
| **rs22115601** | F: CAAATGCCAATAAGCACATGA | SEQ ID NO: 5 | 240 |
| | R: GGGGACAAGTCAATGGTTTC | SEQ ID NO: 6 | |
| **rs220118514** | F: AGGGGACTGACCAAATGTGA | SEQ ID NO: 7 | 189 |
| | R: TTGAAGACAGCAAAGGCAAA | SEQ ID NO: 8 | |
| **rs220118513** | F: AGGGGACTGACCAAATGTGA | SEQ ID NO: 9 | 189 |
| | R: TTGAAGACAGCAAAGGCAAA | SEQ ID NO: 10 | |

| ***CYP1B1* gene** | | | |
|---|---|---|---|
| **rs22594186** | F: CCAAGATCACACACACTGCAA | SEQ ID NO: 11 | 225 |
| | R: GGAAAGAAGGGCAGAGGAAG | SEQ ID NO: 12 | |
| **rs22594187** | F: CCAAGATCACACACACTGCAA | SEQ ID NO: 13 | 225 |
| | R: GGAAAGAAGGGCAGAGGAAG | SEQ ID NO: 14 | |
| **rs22594188** | F: CCAAGATCACACACACTGCAA | SEQ ID NO: 15 | 225 |
| | R: GGAAAGAAGGGCAGAGGAAG | SEQ ID NO: 16 | |

### Polymerase Chain Reaction (PCR) Conditions

The PCR reactions were performed using Thermo Scientific Phusion Green Hot Start II High-Fidelity DNA polymerase, and the following cycles: Initial Denaturation: 98°C for 30 seconds, Denaturation: 98°C for 5 seconds, Annealing: 55°C for 30 seconds, Extension: 72°C for 15 seconds, Final extension: 72°C for 7 seconds, and Hold at 4°C.

*SRBD1 and CYP1B1 PCR Conditions.* For each *SRBD1* SNP and each *CYP1B1* SNP, the reaction mixture contained 6 µl of DNA, 0.3 µl of Phusion Polymerase, 1.5 µl of the corresponding Forward Primer (10 mM), and 1.5 µl of the corresponding Reverse Primer (10 mM), 0.5 µl of 10 mM DNTPs, 5 µl of 5x Phusion Green Buffer, and 10.2 µl of water for a reaction mixture with a total volume of 25 µl.

### Determination of DNA Sequences

The PCR products were electrophoretically separated on a 1% agarose gel, the PCR product bands were cut, and the DNA was extracted from the gels slices using Qiagen MinElute Gel Extraction Kit or Thermo scientific GeneJET Gel extraction Kit, as indicated in the kit experimental guide. The extracted DNA was subjected to cycle sequencing using the Big Dye terminator sequencing kit (Life Technologies, Saint Aubin, France). The sequence cycling reaction products were purified by EDTA/ethanol precipitation.

### Cycle Sequencing Conditions

The cycle sequencing conditions were as follows: 95°C for 10 seconds, 50°C for 30 seconds, 72°C for 4 minutes, and hold at 4°C.

*SRBD1 and CYP1B1 Cycle Sequencing Conditions.* For each *SRBD1* SNP and each *CYP1B1* SNP, the reaction mixture contained 10 µl of DNA, 6 µl of Big Dye Terminator, 3 µl of 5x Sequencing Buffer, and 1 µl of the corresponding Forward Primer for a reaction mixture with a total volume of 20 µl.

### EDTA/Ethanol Precipitation of Amplicons (Cycle-sequencing Products)

10 µl of 125 mM EDTA was added to 20 µl of cycle-sequencing products. To each sample, 200 µl of 100% ethanol were added, and the resulting mixture was incubated at room temperature for 15 minutes, and then centrifuged at 3000g for 30 minutes. The supernatants obtained were immediately discarded, and 500 µl of 70% ethanol were added to each sample. The resulting mixtures were centrifuged at 3000g for 30 minutes. The supernatants obtained were immediately discarded and the tubes were dried using a SpeedVac. After drying, the DNA samples were mixed with high Dye Mix (Life Technologies) and were directly sequenced using an ABI genetic analyzer 3500 (Life Technologies). The sequence data were analyzed using Sequence Analysis 6.

### Example 1: Genotyping of the Two SNPs of the Invention

For each SNP, each sample was classified as: non-risk homozygous, heterozygous, or risk homozygous. The results obtained are presented below.

### 1 - Across Breed Data Analysis

The conditions of analysis were as follows: **Risk Allele:** Manor, **Model:** Multiplicative, **Sig Level:** 0.05.

| | Non risk | Hetero zygous | Risk | Total | risk allele frequency | Statistical Analysis |
|---|---|---|---|---|---|---|
| **rs9172407** | C/C | C/T | T/T | | | |
| Controls | 57 | 19 | 1 | 77 | 13.63% | |
| Glaucomas | 18 | 93 | 24 | 135 | 52.22% | Chi-Square test: P<4.26E-09; Odd Ratio: 3.567 |
| | | | | | | |

| **rs22115601** | C/C | C/T | T/T | | | |
|---|---|---|---|---|---|---|
| Controls | 58 | 16 | 3 | 77 | 14.28% | |
| Glaucomas | 40 | 75 | 20 | 135 | 42.59% | Chi-Square test: P<0.00116827; Odd Ratio: 2.041 |

| **SNP ID** | **x2** | **p-value** | **OR** | **Lower CI of OR** | **Higher CI of OR** | **Significant ?** |
|---|---|---|---|---|---|---|
| **rs9172407** | 34.50 | 4.256E-9 | 3.567 | 2.309 | 5.508 | Y |
| **rs22115601** | 10.54 | 0.001 | 2.041 | 1.322 | 3.15 | Y |

| | | | | | | |
|---|---|---|---|---|---|---|
| Both SNPs (rs9172407 and rs22115601) were found to be significant. | | | | | | |

### 2 - Within Breed Data Analysis

### a) Golden Retriever

The conditions of analysis were as follows: **Risk Allele:** Manor, **Model:** Multiplicative, **Sig Level:** 0.05.

| | Non risk | Hetero zygous | Risk | Total | risk allele frequency | Statistical Analysis |
|---|---|---|---|---|---|---|
| **rs9172407** | C/C | C/T | T/T | | | |
| Controls | 8 | 4 | 1 | 13 | 23.07% | |
| Glaucomas | 0 | 5 | 3 | 8 | 68.75% | Chi-Square test: P<0.003; Odd Ratio: 7.33 |
| | | | | | | |

| **rs22115601** | C/C | C/T | T/T | | | |
|---|---|---|---|---|---|---|
| Controls | 6 | 6 | 1 | 13 | 30.76% | |
| Glaucomas | 1 | 3 | 4 | 8 | 68.75% | Chi-Square test: P<0.0162; Odd Ratio: 4.95 |

| **SNP ID** | **x2** | **p-value** | **OR** | **Lower CI of OR** | **Higher CI of OR** | **Significant ?** |
|---|---|---|---|---|---|---|
| **rs9172407** | 8.576 | 0.003 | 7.333 | 1.815 | 29.631 | Y |
| **rs22115601** | 5.768 | 0.016 | 4.95 | 1.289 | 19.014 | Y |

| | | | | | | |
|---|---|---|---|---|---|---|
| Both SNPs (rs9172407 and rs22115601) were found to be significant in the | | | | | | |

### b) French Bulldog

The conditions of analysis were as follows: **Risk Allele:** Manor, **Model:** Multiplicative, **Sig Level:** 0.05.

| | Non risk | Hetero zygous | Risk | Total | risk allele frequency | Statistical Analysis |
|---|---|---|---|---|---|---|
| **rs9172407** | C/C | C/T | T/T | | | |
| Controls | 11 | 2 | 0 | 13 | 7.69% | |
| Glaucomas | 1 | 12 | 0 | 13 | 46.15% | Chi-Square test: P<0.00177; Odd Ratio: 10.28 |

| **rs22115601** | C/C | C/T | T/T | | | |
|---|---|---|---|---|---|---|
| Controls | 7 | 4 | 2 | 13 | 30.76% | |
| Glaucomas | 1 | 8 | 4 | 13 | 61.53% | Chi-Square test: P<0.026; Odd Ratio: 3.6 |

| **SNP ID** | **x2** | **p-value** | **OR** | **Lower CI of OR** | **Higher CI of OR** | **Significant ?** |
|---|---|---|---|---|---|---|
| **rs9172407** | 9.774 | 0.002 | 10.286 | 2.004 | 52.795 | Y |
| **rs22115601** | 4.952 | 0.026 | 3.6 | 1.142 | 11.347 | Y |

| | | | | | | |
|---|---|---|---|---|---|---|
| Both SNPs (rs9172407 and rs22115601) were found to be significant. | | | | | | |

### c) Eurasier Dogs

The conditions of analysis were as follows: **Risk Allele:** Manor, **Model:** Multiplicative, **Sig Level:** 0.05.

| | Non risk | Hetero zygous | Risk | Total | risk allele frequency | Statistical Analysis |
|---|---|---|---|---|---|---|
| **rs9172407** | C/C | C/T | T/T | | | |
| Controls | 3 | 1 | 0 | 4 | 12.50% | |
| Glaucomas | 1 | 17 | 8 | 26 | 63.46% | Chi-Square test: P<0.0200; Odd Ratio: 0.082 |
| | | | | | | |

| **rs22115601** | C/C | C/T | T/T | | | |
|---|---|---|---|---|---|---|
| Controls | 4 | 0 | 0 | 4 | 0.00% | |
| Glaucomas | 21 | 4 | 1 | 26 | 11.53% | Chi-Square test: P<0.704 Odd Ratio: 0 |

| **SNP ID** | **x2** | **p-value** | **OR** | **Lower CI of OR** | **Higher CI of OR** | **Significant ?** |
|---|---|---|---|---|---|---|
| **rs9172407** | 5.404 | 0.02 | 0.082 | 0.009 | 0.72 | Y |
| **rs22115601** | 0.144 | 0.704 | 0 | 0 | 0 | N |

| | | | | | | |
|---|---|---|---|---|---|---|
| Only SNP rs9172407 was found to be significant. | | | | | | |

Since the number of controls (healthy Eurasiers) in this study was low (only 4), a second study was carried out using samples from the population of 77 healthy dogs described above. The results are presented below.

The conditions of analysis were as follows: **Risk Allele:** Manor, **Model:** Multiplicative, **Sig Level:** 0.05.

| | Non risk | Hetero zygous | Risk | Total | risk allele frequency | Statistical Analysis |
|---|---|---|---|---|---|---|
| **rs9172407** | C/C | C/T | T/T | | | |
| Controls | 59 | 19 | 1 | 77 | 13.63% | |
| Glaucomas | 1 | 17 | 8 | 26 | 63.46% | Chi-Square test: P<1.62E-12; Odd Ratio: 11 |
| | | | | | | |

| **rs22115601** | C/C | C/T | T/T | | | |
|---|---|---|---|---|---|---|
| Controls | 58 | 16 | 3 | 77 | 14.28% | |
| Glaucomas | 21 | 4 | 1 | 26 | 11.54% | Chi-Square test: No significant differences |

| **SNP ID** | **x2** | **p-value** | **OR** | **Lower CI of OR** | **Higher CI of OR** | **Significant ?** |
|---|---|---|---|---|---|---|
| **rs9172407** | 49.89 | 1.62E-12 | 11 | 531 | 22.78 | Y |
| **rs22115601** | 0.25 | 0.617 | 0.783 | 0.299 | 2.05 | N |

| | | | | | | |
|---|---|---|---|---|---|---|
| Only SNP rs9172407 was found to be significant. | | | | | | |

### Example 2: Genotyping of two already known SRBDl SNPs

Two SNPs located in the *SRBD1* gene which have previously been shown (Kanemaki et al., PLoS one, 2013, 8(9): e74372 and US 2014/0141432) to be associated to glaucoma pathology in both Shiba-Inus and Shih-Tzus were also tested here. For each SNP, each sample was classified as: non-risk homozygous, heterozygous, or risk homozygous. The results obtained are presented below.

### 1 - Across Breed Data Analysis

The conditions of analysis were as follows: **Risk Allele:** Manor, **Model:** Multiplicative, **Sig Level:** 0.05.

| | Non risk | Hetero zygous | Risk | Total | risk allele frequency | Statistical Analysis |
|---|---|---|---|---|---|---|
| **rs220118514** | G/G | G/C | C/C | | | |
| Controls | 30 | 21 | 26 | 77 | 47.40% | |
| Glaucomas | 67 | 29 | 39 | 135 | 39.62% | Chi-Square test: P<0.6975; Odd Ratio: 1.087 |
| | | | | | | |

| **rs220118513** | T/T | T/C | C/C | | | |
|---|---|---|---|---|---|---|
| Controls | 1 | 8 | 68 | 77 | 93.50% | |
| Glaucomas | 1 | 15 | 119 | 135 | 93.70% | Chi-Square test: P<0.1952 Odd Ratio: 1.68 |

| **SNP ID** | **x2** | **p-value** | **OR** | **Lower CI of OR** | **Higher CI of OR** | **Significant ?** |
|---|---|---|---|---|---|---|
| **rs220118514** | 0.151 | 0.698 | 1.087 | 0.714 | 1.656 | N |
| **rs220118513** | 1.678 | 0.195 | 1.686 | 0.759 | 3.743 | N |

| | | | | | | |
|---|---|---|---|---|---|---|
| Both SNPs (rs220118514 and rs220118513) were found to be non-significant. | | | | | | |

### 2 - Within Breed Data Analysis

### a) Golden Retriever

The conditions of analysis were as follows: **Risk Allele:** Manor, **Model:** Multiplicative, **Sig Level:** 0.05.

| | Non risk | Hetero zygous | Risk | Total | risk allele frequency | Statistical Analysis |
|---|---|---|---|---|---|---|
| **rs220118514** | G/G | G//C | C/C | | | |
| Controls | 7 | 3 | 3 | 13 | 34.61% | |
| Glaucomas | 5 | 3 | 0 | 8 | 18.75% | Chi-Square test: No statistical differences |
| | | | | | | |

| **rs220118513** | T/T | T/C | C/C | | | |
|---|---|---|---|---|---|---|
| Controls | 0 | 0 | 13 | 13 | 100.00% | |
| Glaucomas | 0 | 0 | 8 | 8 | 100.00% | Chi-Square test: No statistical differences |

| **SNP ID** | **x2** | **p-value** | **OR** | **Lower CI of OR** | **Higher CI of OR** | **Significant ?** |
|---|---|---|---|---|---|---|
| **rs220118514** | 0.568 | 0.451 | 0.436 | 0.098 | 1.94 | N |
| **rs220118513** | - | - | - | - | - | N- |

| | | | | | | |
|---|---|---|---|---|---|---|
| Both SNPs (rs220118514 and rs220118513) were found to be non-significant. | | | | | | |

### b) French Bulldog

The conditions of analysis were as follows: **Risk Allele:** Manor, **Model:** Multiplicative, **Sig Level:** 0.05.

| | Non risk | Hetero zygous | Risk | Total | risk allele frequency | Statistical Analysis |
|---|---|---|---|---|---|---|
| **rs220118514** | G/G | G//C | C/C | | | |
| Controls | 6 | 2 | 5 | 13 | 46.15% | |
| Glaucomas | 7 | 3 | 3 | 13 | 34.61% | Chi-Square test: No statistical differences |
| | | | | | | |

| **rs220118513** | T/T | T/C | C/C | | | |
|---|---|---|---|---|---|---|
| Controls | 0 | 0 | 13 | 13 | 100.00% | |
| Glaucomas | 0 | 1 | 12 | 13 | 96.15% | Chi-Square test: No statistical differences |

| **SNP ID** | **x2** | **p-value** | **OR** | **Lower CI of OR** | **Higher CI of OR** | **Significant ?** |
|---|---|---|---|---|---|---|
| **rs220118514** | 0.719 | 0.397 | 0.618 | 0.202 | 1.887 | N |
| **rs220118513** | - | - | - | - | - | N- |

| | | | | | | |
|---|---|---|---|---|---|---|
| Both SNPs (rs220118514 and rs220118513) were found to be not significant. | | | | | | |

### c) Eurasier Dogs

The conditions of analysis were as follows: **Risk Allele:** Manor, **Model:** Multiplicative, **Sig Level:** 0.05.

| | Non risk | Hetero zygous | Risk | Total | risk allele frequency | Statistical Analysis |
|---|---|---|---|---|---|---|
| **rs220118514** | G/G | G//C | C/C | | | |
| Controls | 1 | 3 | 0 | 4 | 37.50% | |
| Glaucomas | 19 | 7 | 0 | 26 | 13.46% | Chi-Square test: No statistical differences |
| | | | | | | |

| **rs220118513** | T/T | T/C | C/C | | | |
|---|---|---|---|---|---|---|
| Controls | 0 | 0 | 4 | 4 | 100.00% | |
| Glaucomas | 0 | 0 | 26 | 26 | 100.00% | Chi-Square test: No statistical differences |

| **SNP ID** | **x2** | **p-value** | **OR** | **Lower CI of OR** | **Higher CI of OR** | **Significant ?** |
|---|---|---|---|---|---|---|
| **rs220118514** | 1.413 | 0.234 | 0.259 | 0.05 | 1.334 | N |
| **rs220118513** | - | - | - | - | - | N- |

| | | | | | | |
|---|---|---|---|---|---|---|
| Both SNPs (rs220118514 and rs220118513) were found to be non-significant. | | | | | | |

### Example 3: Genotyping of three already known CYP1B1 SNPs

Three SNPs in *CYP1Bi* gene previously shown (Kato et al., Molecular Vision, 2009, 15: 2470) to be associated to glaucoma pathology in Beagles were also tested. In this Example, only the samples from the Golden Retrievers, Jack Russell Terriers, Shar Peis, Border Collies, and Weimaraners from the population of 77 healthy dogs and the population of 135 glaucomatous dogs described above, were studied. For each SNP, each sample was classified as: non-risk homozygous, heterozygous, or risk homozygous. The results obtained are presented below.

### 1 - Across Breed Data Analysis

The conditions of analysis were as follows: **Risk Allele:** Manor, **Model:** Multiplicative, **Sig Level:** 0.05.

| | Non risk | Hetero zygous | Risk | Total | risk allele frequency | Statistical Analysis |
|---|---|---|---|---|---|---|
| **rs22594186** | A/A | A/T | T/T | | | |
| Controls | 7 | 0 | 7 | 14 | 50% | |
| Glaucomas | 17 | 1 | 0 | 18 | 2.77% | Chi-Square test: P<9.685E-6; Odd Ratio: 0.029 |
| | | | | | | |

| **rs22594187** | A/A | A/G | G/G | | | |
|---|---|---|---|---|---|---|
| Controls | 0 | 0 | 14 | 14 | 100.00% | |
| Glaucomas | 0 | 0 | 14 | 18 | 100.00% | Chi-Square test: No significant difference |
| | | | | | | |

| **rs22594188** | C/C | C/T | T/T | | | |
|---|---|---|---|---|---|---|
| Controls | 14 | 0 | 0 | 14. | 0.00% | |
| Glaucomas | 18 | 0 | 0 | 18 | 0.00% | Chi-Square test: No significant difference |

| **SNP ID** | **x2** | **p-value** | **OR** | **Lower CI of OR** | **Higher CI of OR** | **Significant?** |
|---|---|---|---|---|---|---|
| **rs22594186** | 19.572 | 9.685E-6 | 0.029 | 0.003 | 0.238 | Y* |
| **rs22594187** | - | - | - | - | - | N** |
| **rs22594188** | - | - | - | - | - | N** |

| | | | | | | |
|---|---|---|---|---|---|---|
| * In this specific case the control samples presented the mutation for rs220118514 SNP. ** No statistical differences. | | | | | | |

None of the three SNPs (rs22594186, rs22594187, rs22594188) were found to be significant.

### Conclusions

The above Examples clearly demonstrate that *SRBD1* SNPs, rs9172407 and SNP rs22115601, are highly efficient for the diagnosis of glaucoma in a large variety of dog breeds. With these two news SNPs it is now possible to conduct within and across breed canine glaucoma testing. In contrast, the two *SRBD1* SNPs, rs22018514 and rs22018513, described in Kanemaki et al., PLoS one, 2013, 8(9): e74372 and US 2014/0141432) were found to be unable to allow across breed glaucoma testing and within breed glaucoma testing in Golden Retrievers, French Bulldogs and Eurasiers. Similarly the three *CYP1B1* SNPs, rs22594186, rs22594187, rs22594188, described by Kato et al. (Molecular Vision, 2009, 15: 2470) were found to be unable to allow across breed glaucoma testing.

Since the *SRBD1* gene is a susceptibility gene for glaucoma risk, which is shared by dogs and humans (Kanemaki et al., PloS one, 2013, 8(9): e74372), it would be interesting to assess whether SNPs located within the *SRBD1* gene could be useful in the diagnostic of glaucoma in humans.

## Claims

1. An *in vitro* method for identifying a subject at risk of having or developing canine glaucoma, said method comprising a step of:
- determining, in a biological sample obtained from said subject, the identity, presence or absence of a variant allele of single nucleotide polymorphism (SNP) rs22115601, which is located at nucleotide position 401 of the flanking sequence SEQ ID NO: 2.

2. The *in vitro* method according to claim 1, wherein the subject is identified as being at risk of having or developing canine glaucoma if the variant allele of rs22115601 is T at nucleotide position 401 of the flanking sequence SEQ ID NO: 2.

3. An *in vitro* method for identifying a subject at risk of having or developing canine glaucoma, said method comprising a step of:
- determining, in a biological sample obtained from said subject, the identity, presence or absence of a variant allele of a SNP located within the *SRBD1* gene, wherein said SNP is selected from the group consisting of rs9172407, rs22115601, and combination thereof,
wherein the subject belongs to any one of the dog breeds from the group consisting of Akita, Alaskan Malamute, Basset Hound, Beagle, Border Collie, Boston Terrier, Bouvier des Flandres, Brittany Spaniel, Cairn Terrier, Cardigan Welsh, Corgi, Chihuahua, Chow Chow, American Cocker Spaniel, Dachshund, Dalmatian, Great Dane, Dandie Dinmont Terrier, Elkhound, English Cocker Spaniel, English Springer Spaniel, German Shepherd, Giant Schnauzer, Greyhound, Irish Setter, Italian Greyhound, Jack Russell Terrier, Keeshond, Lakeland Terrier, Maltese, Miniature Pinscher, Miniature Schnauzer, Norfolk Terrier, Norwegian Elkhound, Norwich Terrier, Poodle, Samoyed, Scottish Terrier, Sealyham Terrier, Shar Pei, Siberian Husky, Skye Terrier, Smooth Fox Terrier, Tibetan Terrier, Welsh Springer Spaniel, Welsh Terrier, West Highland White Terrier, Wire Fox Terrier, French Bulldog, Boxer, Bichon, German Shepherd, Short-Haired Dachshund, Golden Retriever, Border Collie, Newfoundland, Griffon Vendéen, Australian Shepherd, Long-Haired Dachshund, Groenendael, Doberman, Beauceron, Weimaraner, Bull Terrier, Hovawart, Polish Lowland Sheepdog, Labrador, Great Dane, Pointer, English Setter, Gordon Setter, Korthals Griffon, American Cocker, Jagdterrier, Brittany, Italian Greyhound, Labrador, Pomeranian, Lhassa apso, Cavalier King Charles Spaniel, Tervuren, Bouvier des Flandres, Staffordshire Bull Terrier, Flat-Coated Retriever, Schnauzer, Eurasier,Yorkshire Terrier, Pinscher, Leonberger, and West Highland White Terrier
wherein the subject is identified as being at risk of having or developing canine glaucoma if the variant allele of rs9172407 is T at nucleotide position 401 of the flanking sequence SEQ ID NO: 1 and/or if the variant allele of rs22115601 is T at nucleotide position 401 of the flanking sequence SEQ ID NO: 2.

4. The *in vitro* method according to any one of claims 1 to 3, wherein the subject is a dog which does not exhibit any symptoms of glaucoma.

5. The *in vitro* method according to any one of claims 1 to 4, wherein the biological sample is selected from the group consisting of blood, semen, saliva, tears, urine, fecal material, sweat, buccal cells, skin, hair and other nucleic acid-containing tissue.

6. The *in vitro* method according to claim 5, wherein the biological sample comprises genomic DNA.

7. A reagent for use in a method for identifying a subject at risk of having or developing canine glaucoma, wherein said reagent comprises a pair of primers capable of amplifying at least part of a nucleic acid region containing SNP rs22115601 and optionally a probe capable of specifically hybridizing to at least part of a nucleic acid region containing SNP rs22115601, wherein said pair of primers comprises a forward primer consisting of the sequence set forth in SEQ ID NO: 5 and a reverse primer consisting of the sequence set forth in SEQ ID NO: 6.

8. The reagent for the use according to claim 7, wherein the probe capable of specifically hybridizing to at least part of a nucleic acid region containing SNP rs22115601 can specifically hybridize a nucleic acid region of the flanking sequence SEQ ID NO: 2.

9. The reagent for the use according to claim 7 or claim 8, wherein said reagent further comprises a pair of primers capable of amplifying at least part of a nucleic acid region containing SNP rs9172407 and optionally a probe capable of specifically hybridizing to at least part of a nucleic acid region containing SNP rs9172407, wherein said pair of primers comprises a forward primer consisting of the sequence set forth in SEQ ID NO: 3 and a reverse primer consisting of the sequence set forth in SEQ ID NO: 4.

10. The reagent for the use according to claim 9, wherein the probe capable of specifically hybridizing to at least part of a nucleic acid region containing SNP rs9172407 can specifically hybridize a nucleic acid region of the flanking sequence SEQ ID NO: 1.

11. The reagent for the use according to any one of claims 7 to 10, wherein said probe capable of specifically hybridizing to at least part of a nucleic acid region containing SNP rs22115601 is labeled with a detectable agent or moiety.

12. The reagent for the use according to any one of claims 7 to 11, wherein said probe capable of specifically hybridizing to at least part of a nucleic acid region containing SNP rs22115601 is immobilized on a solid support.

13. The reagent for the use according to claim 9 or claim 10, wherein said probe capable of specifically hybridizing to at least part of a nucleic acid region containing SNP rs9172407 is labeled with a detectable agent or moiety.

14. The reagent for the use according to any one of claims 9, 10 and 13, wherein said probe capable of specifically hybridizing to at least part of a nucleic acid region containing SNP rs9172407 is immobilized on a solid support.

15. A kit for identifying a subject at risk of having or developing canine glaucoma comprising a reagent according to any one of claims 7 to 14.

16. The kit according to claim 15 further comprising instructions for performing an *in vitro* method according to any one of claims 1 to 6.

17. The kit according to claim 15 or claim 16 further comprising amplification reagents.

18. The kit according to any one of claims 15 to 17 further comprising at least one buccal swab brush.

## Patentansprüche

1. *In vitro* Verfahren zum Identifizieren eines Subjekts, das gefährdet ist ein Hunde-Glaukom aufzuweisen oder zu entwickeln, wobei das Verfahren einen Schritt umfasst:
- Bestimmen der Identität, Anwesenheit oder Abwesenheit eines varianten Allels eines Einzelnukleotidpolymorphismus (SNP) rs22115601, das sich an der Nukleotidposition 401 der flankierenden Sequenz SEQ ID NO: 2 befindet, in einer von dem Subjekt erhaltenen biologischen Probe.

2. *In vitro* Verfahren nach Anspruch 1, wobei das Subjekt als gefährdet für das Aufweisen oder Entwickeln eines Hunde-Glaukoms identifiziert wird, falls das variante Allel von rs22115601 T an der Nukleotidposition 401 der flankierenden Sequenz SEQ ID NO: 2 ist.

3. *In vitro* Verfahren zum Identifizieren eines Subjekts, das gefährdet ist ein Hunde-Glaukom aufzuweisen oder zu entwickeln, wobei das Verfahren einen Schritt umfasst:
- Bestimmen der Identität, Anwesenheit oder Abwesenheit eines varianten Allels eines SNP, das sich innerhalb des *SRBD1* Gens befindet, wobei der SNP ausgewählt ist aus der Gruppe bestehend aus rs9172407, rs22115601 und einer Kombination davon, wobei das Subjekt zu einer der Hunderassen gehört aus der Gruppe bestehend aus Akita, Alaskan Malamute, Basset Hound, Beagle, Border Collie, Boston Terrier, Bouvier des Flandres, Brittany Spaniel, Cairn Terrier, Cardigan Welsh, Corgi, Chihuahua, Chow Chow, American Cocker Spaniel, Dackel, Dalmatiner, Dogge, Dandie Dinmont Terrier, Elchhund, Englischer Cocker Spaniel, Englischer Springer Spaniel, Deutscher Schäferhund, Riesenschnauzer, Greyhound, Irish Setter, Italienisches Windspiel, Jack Russell Terrier, Wolfsspitz , Lakeland Terrier, Malteser, Zwergpinscher, Zwergschnauzer, Norfolk Terrier, Norwegischer Elchhund, Norwich Terrier, Pudel, Samojede, Scottish Terrier, Sealyham Terrier, Shar Pei, Siberian Husky, Skye Terrier, Smooth Fox Terrier, Tibet Terrier, Welsh Springer Spaniel, Welsh Terrier, West Highland White Terrier, Wire Fox Terrier, Französische Bulldogge, Boxer, Bichon, Deutscher Schäferhund, Kurzhaardackel, Golden Retriever, Border Collie, Neufundländer, Griffon Vendeen, Australian Shepherd, Langhaardackel, Groenendael, Dobermann, Beauceron, Weimaraner, Bullterrier, Hovawart, Polski Owczarek Nizinny, Labrador, Deutsche Dogge, Pointer, English Setter, Gordon Setter, Korthals Griffon, American Cocker, Jagdterrier, Brittany, Italienisches Windspiel, Labrador, Pomeranian, Lhassa apso, Cavalier King Charles Spaniel, Tervuren, Bouvier des Flandres, Staffordshire Bull Terrier, Flat-Coated Retriever, Schnauzer, Eurasier, Yorkshire Terrier, Pinscher, Leonberger und West Highland White Terrier, wobei das Subjekt als gefährdet identifiziert wird ein Hunde-Glaukom aufzuweisen oder zu entwickeln, wenn das variante Allel von rs9172407 an der Nukleotidposition 401 der flankierenden Sequenz SEQ ID NO: 1 T ist und/oder wenn das variante Allel von rs22115601 an der Nukleotidposition 401 der flankierenden Sequenz SEQ ID NO: 2 T ist.

4. *In vitro* Verfahren nach einem der Ansprüche 1 bis 3, wobei das Subjekt ein Hund ist, der keine Symptome eines Glaukoms zeigt.

5. *In vitro* Verfahren nach einem der Ansprüche 1 bis 4, wobei die biologische Probe ausgewählt ist aus der Gruppe bestehend aus Blut, Samen, Speichel, Tränen, Urin, Stuhlmaterial, Schweiß, Buccalzellen, Haut, Haaren und anderem Nukleinsäureenthaltendem Gewebe.

6. *In vitro* Verfahren nach Anspruch 5, wobei die biologische Probe genomische DNA umfasst.

7. Reagenz zur Verwendung in einem Verfahren zum Identifizieren eines Subjekts, das gefährdet ist ein Hunde-Glaukom aufzuweisen oder zu entwickeln, wobei das Reagenz ein Pimerpaar, das in der Lage ist mindestens einen Teil einer Nukleinsäureregion zu amplifizieren, die SNP rs22115601 enthält, und gegebenenfalls eine Sonde umfasst, die in der Lage ist, spezifisch an mindestens einen Teil einer Nukleinsäureregion zu hybridisieren, die SNP rs22115601 enthält, wobei das Primerpaar einen Forward-Primer, bestehend aus der in SEQ ID NO: 5 angegebene Sequenz, und einen Reverse-Primer, bestehend aus der in SEQ ID NO: 6 angegebene Sequenz, umfasst.

8. Reagenz zur Verwendung nach Anspruch 7, wobei die Sonde, die in der Lage ist, spezifisch an mindestens einen Teil einer Nukleinsäureregion zu hybridisieren, die SNP rs22115601 enthält, spezifisch an eine Nukleinsäureregion der flankierenden Sequenz SEQ ID NO: 2 hybridisieren kann.

9. Reagenz zur Verwendung nach Anspruch 7 oder Anspruch 8, wobei das Reagenz ferner ein Pimerpaar, das in der Lage ist mindestens einen Teil einer Nukleinsäureregion zu amplifizieren, die SNP rs9172407 enthält, und gegebenenfalls eine Sonde umfasst, die in der Lage ist, spezifisch an mindestens einen Teil einer Nukleinsäureregion zu hybridisieren, die SNP rs9172407 enthält, wobei das Primerpaar einen Forward-Primer, bestehend aus der in SEQ ID NO: 3 angegebene Sequenz, und einen Reverse-Primer, bestehend aus der in SEQ ID NO: 4 angegebene Sequenz, umfasst.

10. Reagenz zur Verwendung nach Anspruch 9, wobei die Sonde, die in der Lage ist, spezifisch an mindestens einen Teil einer Nukleinsäureregion zu hybridisieren, die SNP rs9172407 enthält, spezifisch an eine Nukleinsäureregion der flankierenden Sequenz SEQ ID NO: 1 hybridisieren kann.

11. Reagenz zur Verwendung nach einem der Ansprüche 7 bis 10, wobei die Sonde, die in der Lage ist, spezifisch an mindestens einen Teil einer Nukleinsäureregion zu hybridisieren, die SNP rs22115601 enthält, mit einem nachweisbaren Mittel oder Rest markiert ist.

12. Reagenz zur Verwendung nach einem der Ansprüche 7 bis 11, wobei die Sonde, die in der Lage ist, spezifisch an mindestens einen Teil einer Nukleinsäureregion zu hybridisieren, die SNP rs22115601 enthält, auf einem festen Träger immobilisiert ist.

13. Reagenz zur Verwendung nach Anspruch 9 oder Anspruch 10, wobei die Sonde, die in der Lage ist, spezifisch an mindestens einen Teil einer Nukleinsäureregion zu hybridisieren, die SNP rs9172407 enthält, mit einem nachweisbaren Mittel oder Rest markiert ist.

14. Reagenz zur Verwendung nach einem der Ansprüche 9, 10 und 13, wobei die Sonde, die in der Lage ist, spezifisch an mindestens einen Teil einer Nukleinsäureregion zu hybridisieren, die SNP rs9172407 enthält, auf einem festen Träger immobilisiert ist.

15. Kit zum Identifizieren eines Subjekts, das gefährdet ist ein Hundeglaukom aufzuweisen oder zu entwickeln, umfassend ein Reagens nach einem der Ansprüche 7 bis 14.

16. Kit nach Anspruch 15, ferner umfassend Anweisungen zur Durchführung eines *in vitro* Verfahrens nach einem der Ansprüche 1 bis 6.

17. Kit nach Anspruch 15 oder Anspruch 16, ferner umfassend Amplifikationsreagenzien.

18. Kit nach einem der Ansprüche 15 bis 17, ferner umfassend mindestens eine Bukkalabstrichbürste.

## Revendications

1. Procédé *in vitro* d'identification d'un sujet présentant un risque d'avoir ou de développer un glaucome canin, ledit procédé comprenant une étape de :
- détermination, dans un échantillon biologique obtenu dudit sujet, de l'identité, de la présence ou de l'absence d'une variation allélique du polymorphisme mononucléotidique (SNP) rs22115601, qui se trouve au niveau de la position nucléotidique 401 de la séquence flanquante SEQ ID NO : 2.

2. Procédé *in vitro* selon la revendication 1, dans lequel le sujet est identifié comme présentant un risque d'avoir ou de développer un glaucome canin si la variation allélique de rs22115601 est T au niveau de la position nucléotidique 401 de la séquence flanquante SEQ ID NO : 2.

3. Procédé *in vitro* d'identification d'un sujet présentant un risque d'avoir ou de développer un glaucome canin, ledit procédé comprenant une étape de :
- détermination, dans un échantillon biologique obtenu dudit sujet, de l'identité, de la présence ou de l'absence d'une variation allélique d'un SNP situé à l'intérieur du gène SRBD1, dans lequel ledit SNP est sélectionné dans le groupe constitué de rs9172407, rs22115601, et de leur combinaison,
dans lequel le sujet appartient à l'une quelconque des races de chiens du groupe constitué de l'akita, du malamute de l'Alaska, du basset hound, du beagle, du border collie, du boston terrier, du bouvier des Flandres, de l'épagneul breton, du cairn terrier, du welsh cardigan, du corgi, du chihuahua, du chow-chow, de l'épagneul cocker américain, du teckel, du dalmatien, du dogue allemand, du dandie dinmont terrier, de l'elkhound, de l'épagneul cocker anglais, de l'épagneul springer anglais, du berger allemand, du schnauzer géant, du lévrier, du setter irlandais, du petit lévrier italien, du jack russell terrier, du spitz-loup, du lakeland terrier, du maltais, du pinscher miniature, du schnauzer miniature, du norfolk terrier, de l'elkhound norvégien, du terrier de Norwich, du caniche, du samoyède, du terrier écossais, du terrier de Sealyham, du shar-pei, du husky sibérien, du skye terrier, du fox-terrier à poil lisse, du terrier tibétain, welsh springer spaniel, du terrier gallois, du terrier blanc west-highland, du fox-terrier à poil dur, du bouledogue français, du boxer, du bichon, du berger allemand, du teckel à poil court, du golden retriever, du border collie, du terre-neuve, du griffon vendéen, du berger australien, du teckel à poil long, du groenendael, du doberman, du beauceron, du weimaraner, du bull terrier, du hovawart, du berger de plaine polonais, du labrador, du dogue allemand, du pointer, du setter anglais, du setter gordon, du griffon korthals, du cocker américain, du terrier de chasse, de l'épagneul breton, du petit lévrier italien, du labrador, du poméranien, du lhassa apso, de l'épagneul cavalier King Charles, du tervuren, du bouvier des Flandres, du staffordshire bull terrier, du retriever à poil plat, du schnauzer, de l'eurasier, du yorkshier terrier, du pinscher, du léonberger et du terrier blanc west-highland,
dans lequel le sujet est identifié comme présentant un risque d'avoir ou de développer un glaucome canin si la variation allélique de rs9172407 est T au niveau de la position nucléotidique 401 de la séquence flanquante SEQ ID NO: 1 et/ou si la variation allélique de rs22115601 est T au niveau de la position nucléotidique 401 de la séquence flanquante SEQ ID NO : 2.

4. Procédé *in vitro* selon l'une quelconque des revendications 1 à 3, dans lequel le sujet est un chien qui ne présente pas de symptômes de glaucome.

5. Procédé *in vitro* selon l'une quelconque des revendications 1 à 4, dans lequel l'échantillon biologique est sélectionné dans le groupe constitué du sang, du sperme, de la salive, des larmes, de l'urine, de la matière fécale, de la sueur, des cellules buccales, de la peau, des poils ou autre tissu contenant des acides nucléiques.

6. Procédé *in vitro* selon la revendication 5, dans lequel l'échantillon biologique comprend de l'ADN génomique.

7. Réactif pour son utilisation dans un procédé d'identification d'un sujet présentant un risque d'avoir ou de développer un glaucome canin, dans lequel ledit réactif comprend une paire d'amorces capables d'amplifier au moins une partie d'une région d'acide nucléique contenant le SNP rs22115601 et facultativement une sonde capable de spécifiquement s'hybrider à au moins une partie d'une région d'acide nucléique contenant le SNP rs22115601, dans lequel ladite paire d'amorces comprend une amorce sens constituée de la séquence exposée dans SEQ ID NO : 5 et une amorce antisens constituée de la séquence exposée dans SEQ ID NO : 6.

8. Réactif pour son utilisation selon la revendication 7, dans lequel la sonde capable de spécifiquement s'hybrider à au moins une partie d'une région d'acide nucléique contenant le SNP rs22115601 peut spécifiquement s'hybrider à une région d'acide nucléique de la séquence flanquante SEQ ID NO : 2.

9. Réactif pour son utilisation selon la revendication 7 ou la revendication 8, dans lequel ledit réactif comprend en outre une paire d'amorces capables d'amplifier au moins une partie d'une région d'acide nucléique contenant le SNP rs9172407 et facultativement une sonde capable de spécifiquement s'hybrider à au moins une partie d'une région d'acide nucléique contenant le SNP rs9172407, dans lequel ladite paire d'amorces comprend une amorce sens constituée de la séquence exposée dans SEQ ID NO : 3 et une amorce antisens constituée de la séquence exposée dans SEQ ID NO : 4.

10. Réactif pour son utilisation selon la revendication 9, dans lequel la sonde capable de spécifiquement s'hybrider à au moins une partie d'une région d'acide nucléique contenant le SNP rs9172407 peut spécifiquement s'hybrider à une région d'acide nucléique de la séquence flanquante SEQ ID NO : 1.

11. Réactif pour son utilisation selon l'une quelconque des revendications 7 à 10, dans lequel ladite sonde capable de spécifiquement s'hybrider à au moins une partie d'une région d'acide nucléique contenant le SNP rs22115601 est marquée avec un agent ou une fraction détectables.

12. Réactif pour son utilisation selon l'une quelconque des revendications 7 à 11, dans lequel ladite sonde capable de spécifiquement s'hybrider à au moins une partie d'une région d'acide nucléique contenant le SNP rs22115601 est immobilisée sur un support solide.

13. Réactif pour son utilisation selon la revendication 9 ou la revendication 10, dans lequel ladite sonde capable de spécifiquement s'hybrider à au moins une partie d'une région d'acide nucléique contenant le SNP rs9172407 est marquée avec un agent ou une fraction détectables.

14. Réactif pour son utilisation selon l'une quelconque des revendications 9, 10 et 13, dans lequel ladite sonde capable de spécifiquement s'hybrider à au moins une partie d'une région d'acide nucléique contenant le SNP rs9172407 est immobilisée sur un support solide.

15. Kit d'identification d'un sujet présentant un risque d'avoir ou de développer un glaucome canin comprenant un réactif selon l'une quelconque des revendications 7 à 14.

16. Kit selon la revendication 15, comprenant en outre des instructions pour mettre en oeuvre un procédé *in vitro* selon l'une quelconque des revendications 1 à 6.

17. Kit selon la revendication 15 ou la revendication 16 comprenant en outre des réactifs d'amplification.

18. Kit selon l'une quelconque des revendications 15 à 17 comprenant au moins un écouvillon buccal.


### SEQUENCE LISTING

<110> MENICON TCHEDRE, Kissaou et al.
<120> Reagents, Method and Kit for Across and Within Dog Breed Glaucoma Diagnosis
<130> BEP 150529
<160> 16
<170> PatentIn version 3.5
<210> 1
   <211> 801
   <212> DNA
   <213> Artificial Sequence
<220>
   <223> Canis familiaris - flanking sequence for rs9172407
<220>
   <221> misc_feature
   <222> (401)..(401)
   <223> Y is C or T
<400> 1
   gtacaccata tccacactca agatcctgaa tacatctatc atcctctaaa gttttatttg      60
   ctagccaaag ccttgcaaat ctttcttact tttacctccc acaaccagtc tactagtaac     120
   ataacttctt cctccaaaat ataggctaaa tttctgtgat ctccagtgtc tttagtcttc     180
   aatatctctt acccagacta ctgtaatagc ctcttaacta gcttccttgc ttcccctctt     240
   gcctttacta ctcattctcc acataccaat gcaaaaaaaa aagatgattt agaaaatgct     300
   taaccattta atgacttccc actccattct ccacatagaa tcaaatcata tatatttttt     360
   ttttaccaca gccttctaga ataccctaca gaactgaccc ycacctacct ttctaaccac     420
   ctttatcatg tgccataggt cttcagccac agtcactaca ttgtttcagt cactataaca     480
   agtatgatcc tttgccattt caggttcact ctgcctggaa cactatcctc cctgctcatc     540
   aataggatgg ctcctcatct cttaggcctc agcattttag gcctaaaatg ggtttctcag     600
   atatcacatt attataggac cccatttact tccttcatac agtactacac aaagcacaat     660
   ctataattat ccttgtttat tgtctgcatc ccccatgttt actctatggc agtaaactcc     720
   atgaaggaag aaaccacatt tgccttattc acttctcggt aacagagatt agcacttaac     780
   ggctgctcta taactattga t                                               801
<210> 2
   <211> 801
   <212> DNA
   <213> Artificial Sequence
<220>
   <223> canis familiaris - flanking sequence for rs22115601
<220>
   <221> misc_feature
   <222> (401)..(401)
   <223> Y is C or T
<400> 2
   ttttgtttat caaaggatac tatgaaaaaa gtgaaaataa aaccctacag aatggaagaa      60
   attttttata aatcatgtat ctgataaggg gcttacagca atatcatgtt atatataata     120
   tttatatatt ttcttatata ttatatgctt ttaaatatat atgtgtgata tataatatat     180
   aaagaacccc taagatgtag aaaaataatc taaataaaaa gtggtcaggg agcttgaata     240
   gacatttctt caaagaagat ctacaaatgc caataagcac atgaaaagat gctcagtgtt     300
   attatccatg aaggaaatgc aaactaaaac caccctgtga aaccacttta caaccattaa     360
   gatggctata ataaaaaaaa tacagatgaa gaacaagcgg ygatgaggat gtagaaaaac     420
   aaggtttctt gttggggtaa tgaaaatgtt ctaaaactta tcatgatggt ttagaactct     480
   atggaaacca ttgacttgtc cccttaaatg gtgaataata tctcaataaa gctgttagca     540
   aaacccaaac ccaaacaaga ccaagataaa gtttctggag gcaaatttgt gatttcagac     600
   tattttcttt ggttccggct ctattcactg gggccagcag actgtgttag tgaagagttg     660
   gccaaagccc aaaggaactc tcttcatctg tgctttgcct tgagcaattt atttttaaat     720
   aaccctttgt caaattagac atacatagtc tggttttgca tatcctctcc tagggttttt     780
   gatgttttta tcttggggat g                                               801
<210> 3
   <211> 20
   <212> DNA
   <213> Artificial Sequence
<220>
   <223> forward primer for rs9172407
<400> 3
   cttccttgct tcccctcttg 20
<210> 4
   <211> 20
   <212> DNA
   <213> Artificial Sequence
<220>
   <223> reverse primer for rs9172407
<400> 4
   gaacctgaaa tggcaaagga 20
<210> 5
   <211> 21
   <212> DNA
   <213> Artificial Sequence
<220>
   <223> forward primer for rs22115601
<400> 5
   caaatgccaa taagcacatg a 21
<210> 6
   <211> 20
   <212> DNA
   <213> Artificial Sequence
<220>
   <223> reverse primer for rs22115601
<400> 6
   ggggacaagt caatggtttc 20
<210> 7
   <211> 20
   <212> DNA
   <213> Artificial Sequence
<220>
   <223> forward primer for rs220118514
<400> 7
   aggggactga ccaaatgtga 20
<210> 8
   <211> 20
   <212> DNA
   <213> Artificial Sequence
<220>
   <223> reverse primer for rs220118514
<400> 8
   ttgaagacag caaaggcaaa 20
<210> 9
   <211> 20
   <212> DNA
   <213> Artificial Sequence
<220>
   <223> forward primer for rs220118513
<400> 9
   aggggactga ccaaatgtga 20
<210> 10
   <211> 20
   <212> DNA
   <213> Artificial Sequence
<220>
   <223> reverse primer for rs220118513
<400> 10
   ttgaagacag caaaggcaaa 20
<210> 11
   <211> 21
   <212> DNA
   <213> Artificial Sequence
<220>
   <223> forward primer for rs22594186
<400> 11
   ccaagatcac acacactgca a 21
<210> 12
   <211> 20
   <212> DNA
   <213> Artificial Sequence
<220>
   <223> reverse primer for rs22594186
<400> 12
   ggaaagaagg gcagaggaag 20
<210> 13
   <211> 21
   <212> DNA
   <213> Artificial Sequence
<220>
   <223> forward primer for rs22594187
<400> 13
   ccaagatcac acacactgca a 21
<210> 14
   <211> 20
   <212> DNA
   <213> Artificial Sequence
<220>
   <223> reverse primer for rs22594187
<400> 14
   ggaaagaagg gcagaggaag 20
<210> 15
   <211> 21
   <212> DNA
   <213> Artificial Sequence
<220>
   <223> forward primer for rs22594188
<400> 15
   ccaagatcac acacactgca a 21
<210> 16
   <211> 20
   <212> DNA
   <213> Artificial Sequence
<220>
   <223> reverse primer for rs22594188
<400> 16
   ggaaagaagg gcagaggaag 20